# EUROPEAN PATENT SPECIFICATION

(11) **EP 2 504 003 B1**
(45) Date of publication and mention of the grant of the patent: **09.11.2016**
(21) Application number: 10784416.9
(22) Date of filing: 23.11.2010
(51) Int. Cl.: A61K 31/18, A61K 9/00, A61P 9/12

(54) **NITROXYL DONORS FOR THE TREATMENT OF PULMONARY HYPERTENSION**
NITROXYLDONATOREN ZUR BEHANDLUNG VON LUNGENHOCHDRUCK
DONNEURS DE NITROXYLE DESTINÉS AU TRAITEMENT DE L'HYPERTENSION PULMONAIRE

(30) Priority: 23.11.2009 US 263698 P; 24.11.2009 US 264129 P
(43) Date of publication of application: 03.10.2012
(73) Proprietor: Cardioxyl Pharmaceuticals, Inc., Chapel Hill, NC 27517 (US)
(72) Inventor: KALISH, Vincent J., Annapolis, Maryland 21401 (US); MAZHARI, Reza, Towson, Maryland 21204 (US)
(74) Representative: Wright, Simon Mark
(86) International application number: PCT/US2010/057844
(87) International publication number: WO 2011/063400

(56) References cited:
- WO-A1-98/19996
- WO-A1-2007/002444
- WO-A1-2007/109175
- WO-A1-2009/042970
- WO-A1-2009/137717
- FR-A1- 2 832 634
- MORRIS ET AL: "Exogenous inhaled nitric oxide as a selective pulmonary vasodilator", SEMINARS IN ANESTHESIA, SAUNDERS, CO, NEW YORK, NY, US, vol. 15, no. 1, 1 March 1996 (1996-03-01), pages 47-60, XP005451460, ISSN: 0277-0326, DOI: DOI:10.1016/S0277-0326(96)80006-7

## Description

Pulmonary hypertension (PH) is a generic term for a group of conditions characterized by elevated blood pressure in the arteries of the lungs (pulmonary arteries). In patients with PH, characteristic changes occur within the pulmonary circulation. These changes include thickening of the linings and obstruction of the small pulmonary blood vessels. As a result of these changes, pressure in the pulmonary circulation rises, and resistance in the blood flowing through the vessels increases. This increased resistance puts a strain on the right side of the heart as it must work harder to pump blood to the lungs. This strain can cause the heart to enlarge. Eventually, heart failure can develop.

The World Health Organization (WHO) classification of PH¹, as updated in the 2008 4^{th} World Conference in Dana Point, California, includes five groups.

WHO Group 1 represents pulmonary arterial hypertension (PAH). PAH is a particularly progressive form of PH characterized by narrowing of the precapillary pulmonary arteries. Obstruction of these precapillary pulmonary arteries leads to increased pulmonary vascular resistance and potentially right heart failure and premature death. Clinical characteristics of PAH include persistently elevated mean pulmonary arterial pressure (MPAP), in combination with normal pulmonary capillary wedge pressure (PCWP) and elevated pulmonary vascular resistance (PVR). The diagnosis of PAH may include clinical parameters that extend beyond these hemodynamic measurements, including precapillary PH, pulmonary hypertensive arteriopathy (usually with plexiform lesions), slow clinical onset (months, years) and a chronic time course (years) characterized by progressive deterioration.
¹ The initial attempt to develop a classification for PH was undertaken during the WHO Conference on PH in 1973. Since then, the PH classification has been revised three times, first at the 1998 2^{nd} World Symposium in Evian, France, then at the 2003 3^{rd} World Symposium in Venice, Italy, and most recently at the 2008 4th World Symposium in Dana Point, California.

WHO Group 2 represents PH owing to left heart disease. Types of left heart disease include systolic dysfunction, diastolic dysfunction and valvular disease.

Other WHO groups include PH owing to lung diseases and/or hypoxia (Group 3), chronic thromboembolic PH (Group 4), and PH with unclear multifactorial mechanisms (Group 5).

Notwithstanding the current WHO classification, some literature still refer to the older classification system of "primary" and "secondary" PH. Primary PH refers to idiopathic PH, while secondary PH refers to PH that develops from another medical condition. For example, under the older classification system, PH owing to left heart disease was classified as PH secondary to left heart disease.

Current therapies for PH include supplemental oxygen, diuretics, oral vasodilators such as calcium channel blockers, anticoagulants, inotropic agents, prostanoids, endothelin receptor antagonists, and phosphodiesterase type-5 inhibitors. While such therapies have met with some success, many PH patients fail to respond to these therapies. Thus, a need remains for additional safe and effective treatments for PH.

### Brief Description of the Drawings

FIG. 1 shows the intravenous effects of a nitroxyl (HNO) donor on mean and systolic (peak) pulmonary artery pressure (PAP) in rats.
FIG. 2 shows the intravenous effects of a nitroxyl (HNO) donor on mean arterial pressure (MPAP) and heart rate in rats.

### Definitions

Unless clearly indicated otherwise, the following terms as used herein have the meanings indicated below.

"A", "an" and the like refers to one or more.

"Aralkyl" refers to a residue in which an aryl moiety is attached to the parent structure via an alkyl residue. Examples include benzyl (-CH₂-Ph), phenethyl (-CH₂CH₂Ph), phenylvinyl (-CH=CH-Ph), phenylallyl and the like.

"Acyl" refers to and includes the groups -C(O)H, -C(O)alkyl, -C(O)substituted alkyl, -C(O)alkenyl, -C(O)substituted alkenyl, -C(O)alkynyl, -C(O)substituted alkynyl, - C(O)cycloalkyl, -C(O)substituted cycloalkyl, -C(O)aryl, -C(O)substituted aryl, - C(O)heteroaryl, -C(O)substituted heteroaryl, -C(O)heterocyclic, and -C(O)substituted heterocyclic wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein or otherwise known in the art.

"Acylamino" refers to the group -C(O)NRₐR_{b} wherein each Rₐ and R_{b} group is independently selected from hydrogen, alkyl, substituted alkyl, alkenyl, substituted alkenyl, alkynyl, substituted alkynyl, aryl, substituted aryl, heteroaryl, substituted heteroaryl, heterocyclic, and substituted heterocyclic, or Rₐ and R_{b} groups can be joined together with the nitrogen atom to form a heterocyclic or substituted heterocyclic ring. An example of an acylamino moiety is -C(O)morpholino.

"Heterocyclyl" or "heterocycloalkyl" refers to a cycloalkyl residue in which one to four of the carbons is replaced by a heteroatom such as oxygen, nitrogen or sulfur. Examples of heterocycles whose radicals are heterocyclyl groups include tetrahydropyran, morpholine, pyrrolidine, piperidine, thiazolidine, oxazole, oxazoline, isoxazole, dioxane, tetrahydrofuran and the like. A specific example of a heterocyclyl residue is tetrahydropyran-2-yl.

"Substituted heterocylyl" or "substituted heterocylcoalkyl" refers to an heterocyclyl group having from 1 to 5 substituents. For instance, a heterocyclyl group substituted with 1 to 5 groups such as halo, nitro, cyano, oxo, aryl, alkoxy, alkyl, acyl, acylamino, amino, hydroxyl, carboxyl, carboxyalkyl, thiol, thioalkyl, heterocyclyl, -OS(O)₂-alkyl, and the like is a substituted alkyl. A particular example of a substituted heterocylcoalkyl is N-methylpiperazino.

"Alkyl" intends linear hydrocarbon structures having 1 to 20 carbon atoms, 1 to 12 carbon atoms or 1 to 8 carbon atoms. Alkyl groups of fewer carbon atoms are embraced, such as so-called "lower alkyl" groups having 1 to 4 carbon atoms. "Alkyl" also intends branched or cyclic hydrocarbon structures having 3 to 20 carbon atoms, 3 to 12 carbon atoms and 3 to 8 carbon atoms. For any use of the term "alkyl," unless clearly indicated otherwise, it is intended to embrace all variations of alkyl groups disclosed herein, as measured by the number of carbon atoms, the same as if each and every alkyl group was explicitly and individually listed for each usage of the term. For instance, when a group such as R³ may be an "alkyl," intended is a C₁-C₂₀ alkyl or a C₁-C₁₂ alkyl or a C₁-C₈ alkyl or a lower alkyl or a C₂-C₂₀ alkyl or a C₃-C₁₂ alkyl or a C₃-C₈ alkyl. The same is true for other groups listed herein, which may include groups under other definitions, where a certain number of atoms is listed in the definition. When the alkyl group is cyclic, it may also be referred to as a cycloalkyl group and have, for example, 1 to 20 annular carbon atoms, 1 to 12 annular carbon atoms and 1 to 8 annular carbon atoms. When an alkyl residue having a specific number of carbons is named, all geometric isomers having that number of carbons are intended to be encompassed; thus, for example, "butyl" is meant to include n-butyl, sec-butyl, iso-butyl and t-butyl; "propyl" includes n-propyl and iso-propyl. Examples of alkyl groups include methyl, ethyl, n-propyl, i-propyl, t-butyl, n-heptyl, octyl, cyclopentyl, cyclopropyl, cyclobutyl, norbornyl, and the like. One or more degrees of unsaturation may occur in an alkyl group. Thus, an alkyl group also embraces alkenyl and alkynyl residues. "Alkenyl" refers to a group of 2 or more carbon atoms, such as 2 to 10 carbon atoms and 2 to 6 carbon atoms, having at least 1, in some cases 1 to 2, sites of alkenyl unsaturation. Examples of an alkenyl group include -C=CH₂, -CH₂CH=CHCH₃ and -CH₂CH=CH-CH=CH₂. "Alkynyl" refers to a group of 2 or more carbon atoms, such as 2 to 10 carbon atoms and 3 to 6 carbon atoms, having at least 1, in some cases 1 to 2, sites of alkynyl unsaturation, such as the moiety -C=CH. Alkyl is also used herein to denote an alkyl residue as part of a larger functional group and when so used, is taken together with other atoms to form another functional group. For instance, "-C(O)Oalkyl" refers to an ester functional group, where the alkyl portion of the moiety may be any alkyl group; nonlimiting examples include -C(O)OCH₃, -C(O)(O)CH=CH₂ and the like. Another example of an alkyl group as part of a larger structure includes the residue - NHC(O)alkylC(O)OH, which, for example, may be NHC(O)CH₂CH₂C(O)OH when alkyl is -CH₂CH₂-.

"Substituted alkyl" refers to an alkyl group having from 1 to 5 substituents. For instance, an alkyl group substituted with a group such as halo, nitro, cyano, oxo, aryl, alkoxy, acyl, acylamino, amino, hydroxyl, carboxyl, carboxylalkyl, thiol, thioalkyl, heterocyclyl, - OS(O)₂-alkyl, and the like is a substituted alkyl. Likewise, "substituted alkenyl" and "substituted alkynyl" refer to alkenyl or alkynyl groups having 1 to 5 substituents.

"Substituted" means that a hydrogen radical on a compound or group (such as, for example, alkyl, substituted alkyl, alkenyl, substituted alkenyl, alkynyl, substituted alkynyl, aryl, substituted aryl, aralkyl, substituted aralkyl, heteroaryl, substituted heteroaryl, heteroaralkyl, substituted heteroaralkyl, cycloalkyl, substituted cycloalkyl, heterocycloalkyl, substituted heterocycloalkyl, heterocyclyl and substituted heterocyclyl) is replaced with a group (the "substituent") that does not substantially adversely affect the stability of the compound. In some embodiments, the substituents are those which do not adversely affect the activity of a compound. The term "substituted" refers to one or more substituents (which may be the same or different), each replacing a hydrogen atom. Examples of substituents include, but are not limited to, halo (F, Cl, Br, or I), hydroxyl, amino, alkylamino, arylamino, dialkylamino, diarylamino, cyano, nitro, mercapto, oxo, carbonyl, thio, imino, formyl, carbamido, carbamyl, carboxyl, thioureido, thiocyanato, sulfoamido, sulfonylalkyl, sulfonylaryl, alkyl, alkenyl, alkoxy, mercaptoalkoxy, aryl, heteroaryl, cyclyl, heterocyclyl, wherein alkyl, alkenyl, alkyloxy, aryl, heteroaryl, cyclyl, and heterocyclyl are optionally substituted with alkyl, aryl, heteroaryl, halogen, hydroxyl, amino, mercapto, cyano, nitro, oxo (=O), thioxo (=S), or imino (=Nalkyl). In some embodiments, substituents on any group (such as, for example, alkyl, substituted alkyl, alkenyl, substituted alkenyl, alkynyl, substituted alkynyl, aryl, substituted aryl, aralkyl, substituted aralkyl, heteroaryl, substituted heteroaryl, heteroaralkyl, substituted heteroaralkyl, cycloalkyl, substituted cycloalkyl, heterocycloalkyl, substituted heterocycloalkyl, heterocyclyl and substituted heterocyclyl) are at any atom of that group (such as on a carbon atom of the primary carbon chain of a substituted alkyl group or on a substituent already present on a substituted alkyl group), wherein any group that can be substituted (such as, for example, alkyl, alkenyl, alkynyl, aryl, aralkyl, heteroaryl, heteroaralkyl, cycloalkyl, cyclyl, heterocycloalkyl, and heterocyclyl) can be optionally substituted with one or more substituents (which may be the same or different), each replacing a hydrogen atom. Examples of substituents include, but not limited to alkyl, alkenyl, alkynyl, cyclyl, cycloalkyl, heterocyclyl, heterocycloalkyl, aralkyl, heteroaralkyl, aryl, heteroaryl, halo, haloalkyl, cyano, nitro, alkoxy, aryloxy, hydroxyl, hydroxylalkyl, oxo, carbonyl, carboxyl, formyl, alkylcarbonyl, alkylcarbonylalkyl, alkoxycarbonyl, alkylcarbonyloxy, aryloxycarbonyl, heteroaryloxy, heteroaryloxycarbonyl, thio, mercapto, mercaptoalkyl, arylsulfonyl, amino, aminoalkyl, dialkylamino, alkylcarbonylamino, alkylaminocarbonyl, or alkoxycarbonylamino; alkylamino, arylamino, diarylamino, alkylcarbonyl, or arylamino-substituted aryl; arylalkylamino, aralkylaminocarbonyl, amido, alkylaminosulfonyl, arylaminosulfonyl, dialkylaminosulfonyl, alkylsulfonylamino, arylsulfonylamino, imino, carbamido, carbamyl, thioureido, thiocyanato, sulfoamido, sulfonylalkyl, sulfonylaryl, or mercaptoalkoxy. Additional examples of substituents include, without limitation, halo, CN, NO₂, OR¹¹, SR¹¹, S(O)₂OR¹¹, NR¹¹R¹², C₁-C₂perfluoroalkyl, C₁-C₂ perfluoroalkoxy, 1,2- methylenedioxy, (=O), (=S), (=NR¹¹), C(O)OR¹¹, C(O)R¹¹R¹², OC(O)NR¹¹R¹², NR¹¹C(O)NR¹¹R¹², C(NR¹²)NR¹¹R¹², NR¹¹C(NR¹²)NR¹¹R¹², S(O)₂NR¹¹R¹²R¹³, C(O)H, C(O)R¹³, NR¹¹C(O)R¹³, Si(R¹¹)₃, OSi(R¹¹)₃, Si(OH)₂R¹¹, B(OH)₂, P(O)(OR¹¹)₂, S(O)R¹³, and S(O)₂R¹³. Each R¹¹ is independently hydrogen, C₁-C₆ alkyl optionally substituted with cycloalkyl, aryl, heterocyclyl, or heteroaryl. Each R¹² is independently hydrogen, C₃-C₆ cycloalkyl, aryl, heterocyclyl, heteroaryl, C₁-C₄ alkyl or C₁-C₄ alkyl substituted with C₃-C₆ cycloalkyl, aryl, heterocyclyl or heteroaryl. Each R¹³ is independently C₃-C₆ cycloalkyl, aryl, heterocyclyl, heteroaryl, C₁-C₄ alkyl or C₁-C₄ alkyl substituted with C₃-C₆ cycloalkyl, aryl, heterocyclyl or heteroaryl. Each C₃-C₆ cycloalkyl, aryl, heterocyclyl, heteroaryl and C₁-C₄ alkyl in each R¹¹, R¹² and R¹³ can optionally be substituted with halo, CN, C₁- C₄ alkyl, OH, C₁-C₄ alkoxy, COOH, C(O)OC₁-C₄ alkyl, NH₂, C₁-C₄ alkylamino, or C₁-C₄ dialkylamino. Substituents can also be "electron-withdrawing groups."

"Electron withdrawing group" refers to a group that reduces electron density of the moiety to which it is attached (relative to the density of the moiety without the substituent). Examples include, without limitation, F, Cl, Br, I, -CN, -CF₃, -NO₂, -SH, -C(O)H, - C(O)alkyl, -C(O)Oalkyl, -C(O)OH, -C(O)Cl, -S(O)₂OH, -S(O)₂NHOH, -NH₃ and the like.

"Halo" refers to fluoro, chloro, bromo or iodo.

"Alkylsulfonyl" refers to a group selected from -SO₂alkyl and -SO₂substituted alkyl, which includes the residues -SO₂cycloalkyl, -SO₂substituted cycloalkyl, -SO₂alkenyl, - SO₂substituted alkenyl, -SO₂alkynyl, and -SO₂substituted alkynyl, where alkyl, substituted alkyl, alkenyl, substituted alkenyl, alkynyl, substituted alkynyl, cycloalkyl and substituted cycloalkyl are as defined herein.

"N-hydroxylsulfonamidyl" refers to -S(O)₂NROH, where R is H or alkyl.

"Perhaloalkyl" refers to an alkyl group where each H of the hydrocarbon is replaced with F. Examples of perhalo groups include -CF₃ and -CF₂CF₃.

"Aryl" refers to a monocyclic, bicyclic or tricyclic aromatic ring radical. In some embodiments, an aryl group is a 5- or 6-membered aromatic or heteroaromatic ring containing 0-3 annular heteroatoms selected from O, N and S; a bicyclic 9- or 10-membered aromatic or heteroaromatic ring system (meaning the ring system has 9 or 10 annular atoms) containing 0-3 annular heteroatoms selected from O, N and S; or a tricyclic 13- or 14-membered aromatic or heteroaromatic ring system (meaning the ring system has 13 or 14 annular atoms) containing 0-3 annular heteroatoms selected from O, N, or S. Examples of aryl radicals include, for example, phenyl, naphthalenyl, indanyl, tetralinyl, imidazolyl, pyridinyl, indolyl, thiophenyl, benzopyranonyl, thiazolyl, furanyl, benzimidazolyl, benzoxazolyl, benzthiazolyl, quinolinyl, isoquinolinyl, quinoxalinyl, pyrimidinyl, pyrazinyl, tetrazolyl and pyrazolyl.

"Substituted aryl" refers to a group having from 1 to 3 substituents. For instance, an aryl group substituted with 1 to 3 groups, such as halo, nitro, cyano, oxo, aryl, alkoxy, alkyl, acyl, acylamino, amino, hydroxyl, carboxyl, carboxylalkyl, thiol, thioalkyl, heterocyclyl, - OS(O)₂-alkyl and the like, is a substituted aryl.

"Alkoxy" refers to an alkyl group that is connected to the parent structure through an oxygen atom (-O-alkyl). When a cycloalkyl group is connected to the parent structure through an oxygen atom, the group may also be referred to as a cycloalkoxy group. Examples include methoxy, ethoxy, propoxy, isopropoxy, cyclopropyloxy, cyclohexyloxy and the like. A "perhaloalkoxy" intends a perhaloalkyl group attached to the parent structure through an oxygen, such as the residue -O-CF₃.

"Aryloxy" refers to an aryl group that is connected to the parent structure through an oxygen atom (-O-aryl), which by way of example includes the residues phenoxy, naphthoxy, and the like. "Substituted aryloxy" refers to a substituted aryl group connected to the parent structure through an oxygen atom (-O-substituted aryl).

"Alkylsulfanyl" refers to an alkyl group that is connected to the parent structure through a sulfur atom (-S-alkyl) and refers to groups -S-alkyl and -S-substituted alkyl, which include the residues -S-cycloalkyl, -S-substituted cycloalkyl, -S-alkenyl, -S-substituted alkenyl, -S-alkynyl, and -S-substituted alkynyl, where alkyl, substituted alkyl, alkenyl, substituted alkenyl, alkynyl, substituted alkynyl, cycloalkyl and substituted cycloalkyl are as defined herein. When a cycloalkyl group is connected to the parent structure through an sulfur atom, the group may also be referred to as a cycloalkylsulfanyl group. By way of example, alkylsulfanyl includes -S-CH(CH₃), -S-CH₂CH₃ and the like.

"Alkylsulfinyl" refers to an alkyl group that is connected to the parent structure through a S(O) moiety and refers to groups -S(O)alkyl and -S(O)substituted alkyl, which includes the residues -S(O)cycloalkyl, -S(O)substituted cycloalkyl, -S(O)alkenyl, - S(O)substituted alkenyl, -S(O)alkynyl, -S(O)substituted alkynyl, where alkyl, substituted alkyl, alkenyl, substituted alkenyl, alkynyl, substituted alkynyl, cycloalkyl and substituted cycloalkyl are as defined herein. By way of example, alkylsulfinyl includes the residues - S(O)CH(CH₃), -S(O)CH₃, -S(O)cyclopentane and the like.

"Arylsulfinyl" refers to an aryl group that is connected to the parent structure through a S(O) moiety, which by way of example includes the residue -S(O)Ph.

"Dialkylamino" refers to -NR₂ where each R is an alkyl group. Examples of dialkylamino groups include -N(CH₃)₂, -N(CH₂CH₂CH₂CH₃)₂, and N(CH₃)(CH₂CH₂CH₂CH₃).

"Carboxyl" refers to -C(O)OH.

"Carboxyl ester" refers to a group selected from -C(O)O-alkyl, -C(O)O-substitued alkyl, -C(O)O-aryl, -C(O)O-substituted aryl, -C(O)O-alkenyl, -C(O)O-substituted alkenyl, - C(O)O-alkynyl, -C(O)O-substituted alkynyl, -C(O)O-heteroaryl, -C(O)O-substitued heteroaryl, -C(O)O-heterocyclic and -C(O)O-substitued heterocyclic.

"Sulfonylamino" refers to a group selected from -SO₂NH₂, -SO₂NR-alkyl, -SO₂NR-substituted alkyl, -SO₂NR-alkenyl, -SO₂NR-substituted alkenyl, -SO₂NR-alkynyl, -SO₂NR-substituted alkynyl, -SO₂NR-aryl, -SO₂NR-substituted aryl, -SO₂NR-heteroaryl, -SO₂NR-substituted heteroaryl, -SO₂NR-heterocyclic and -SO₂NR-substituted heterocyclic wherein R is hydrogen or alkyl, or -SO₂NR₂, wherein the two R groups are taken together and with the nitrogen atom to which they are attached to form a heterocyclic or substituted heterocyclic ring.

"Carbonylamino" refers to a group selected from -CONH₂, -CONR-alkyl, -CONR-substituted alkyl, -CONR-alkenyl, -CONR-substituted alkenyl, -CONR-alkynyl, -CONR-substituted alkynyl, -CONR-aryl, -CONR-substituted aryl, -CONR-heteroaryl, -CONR-substituted heteroaryl, -CONR-heterocyclic, and -CONR-substituted heterocyclic wherein R is hydrogen or alkyl, or -CONR₂, where the two R groups are taken together and with the nitrogen atom to which they are attached to form a heterocyclic or substituted heterocyclic ring.

"Optional" or "optionally" means that the subsequently described event or circumstance may or may not occur, and that the description includes instances where the event or circumstance occurs and instances in which it does not. For example, an alkyl that is "optionally substituted" encompasses both an alkyl that is unsubstituted and an alkyl that is substituted.

"Pharmaceutically acceptable salt" refers to pharmaceutically acceptable salts of a compound described herein, such as a compound of Formula (I), (II), (III) or (IV) or other nitroxyl donors, which salts may be derived from a variety of organic and inorganic counter ions well known in the art and include, by way of example, sodium, potassium, calcium, magnesium, ammonium, tetraalkylammonium, and the like; when the molecule contains a basic functionality, salts of organic or inorganic acids, such as hydrochloride, hydrobromide, tartrate, mesylate, acetate, maleate, oxalate and the like. Illustrative salts include, but are not limited, to sulfate, citrate, acetate, chloride, bromide, iodide, nitrate, bisulfate, phosphate, acid phosphate, lactate, salicylate, acid citrate, tartrate, oleate, tannate, pantothenate, bitartrate, ascorbate, succinate, maleate, besylate, fumarate, gluconate, glucaronate, saccharate, formate, benzoate, glutamate, methanesulfonate, ethanesulfonate, benzenesulfonate, and p- toluenesulfonate salts. Accordingly, a salt may be prepared from a compound of any one of the formulae disclosed herein having an acidic functional group, such as a carboxylic acid functional group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N- methyl,N-ethylamine; diethylamine; triethylamine; mono-, bis-, or tris-(2-hydroxy- lower alkyl amines), such as mono-, bis-, or tris-(2-hydroxyethyl)amine, 2-hydroxy- tert-butylamine, or tris-(hydroxymethyl)methylamine, N,N,-di-lower alkyl-N-(hydroxy lower alkyl)-amines, such as N,N-dimethyl-N-(2-hydroxyethyl) amine, or tri-(2-hydroxyethyl)amine; N-methyl-D-glucamine; and amino acids such as arginine, lysine, and the like. A salt may also be prepared from a compound of any one of the formulae disclosed herein having a basic functional group, such as an amino functional group, and a pharmaceutically acceptable inorganic or organic acid. Suitable acids include hydrogen sulfate, citric acid, acetic acid, hydrochloric acid (HCl), hydrogen bromide (HBr), hydrogen iodide (HI), nitric acid, phosphoric acid, lactic acid, salicylic acid, tartaric acid, ascorbic acid, succinic acid, maleic acid, besylic acid, fumaric acid, gluconic acid, glucaronic acid, formic acid, benzoic acid, glutamic acid, methanesulfonic acid, ethanesulfonic acid, benzenesulfonic acid, and p-toluenesulfonic acid.

"Pharmaceutically acceptable" refers to those properties and/or substances that are acceptable to the patient from a pharmacological and/or toxicological point of view, and/or to the manufacturing pharmaceutical chemist from a physical and/or chemical point of view regarding composition, formulation, stability, patient acceptance, bioavailability and compatibility with other ingredients.

"Pharmaceutically acceptable excipient" refers to any substance, not itself a therapeutic agent, used as a carrier, diluent, adjuvant, binder, and/or vehicle for delivery of a therapeutic agent to a patient, or added to a pharmaceutical composition to improve its handling or storage properties or to permit or facilitate formation of a compound or composition into a unit dosage form for administration. Pharmaceutically acceptable excipients are well known in the pharmaceutical arts and are described, for example, in Remington's Pharmaceutical Sciences, Mack Publishing Co., Easton, Pa (e.g., 20th Ed., 2000), and Handbook of Pharmaceutical Excipients, American Pharmaceutical Association, Washington, D.C., (e.g., 1st, 2nd and 3rd Eds., 1986, 1994 and 2000, respectively). As will be known to those skilled in the art, pharmaceutically acceptable excipients may provide a variety of functions and may be described as wetting agents, buffering agents, suspending agents, lubricating agents, emulsifiers, disintegrants, absorbents, preservatives, surfactants, colorants, flavorants, and sweeteners. Examples of pharmaceutically acceptable excipients include without limitation: (1) sugars, such as lactose, glucose and sucrose; (2) starches, such as corn starch and potato starch; (3) cellulose and its derivatives, such as sodium carboxymethyl cellulose, ethyl cellulose, cellulose acetate, hydroxypropylmethylcellulose, and hydroxypropylcellulose; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

"Unit dosage form" refers to a physically discrete unit suitable as a unitary dosage for human or other animal patients. Each unit dosage form may contain a predetermined amount of an active substance (*e*.*g*., HNO donor) calculated to produce a desired effect.

Unless clearly indicated otherwise, an "individual" or "patient" refers to an animal, such as a mammal, including but not limited, to a human. Hence, the methods described herein can be useful in human therapy and veterinary applications. In some embodiments, the individual or patient is a mammal. In some embodiments, the individual or patient is a human.

"Effective amount" refers to such amount of a compound or a pharmaceutically acceptable salt thereof, which in combination with its parameters of efficacy and toxicity, as well as based on the knowledge of the practicing specialist should be effective in a given therapeutic form. As is understood in the art, an effective amount may be in one or more doses.

"Treatment" or "treating" is an approach for obtaining a beneficial or desired result, including clinical results. For purposes of this invention, beneficial or desired results include but are not limited to inhibiting and/or suppressing the onset and/or development of a disease or condition or reducing the severity of such disease or condition, such as reducing the number and/or severity of symptoms associated with the disease or condition, increasing the quality of life of those suffering from the disease or condition, decreasing the dose of other medications required to treat the disease or condition, enhancing the effect of another medication an individual is taking for the disease or condition, and prolonging survival of individuals having the disease or condition.

"Preventing" refers to reducing the probability of developing a disorder or condition in an individual who does not have, but is at risk of developing a disorder or condition. An individual "at risk" may or may not have a detectable disease or condition, and may or may not have displayed a detectable disease or condition prior to the treatment methods described herein. "At risk" denotes that an individual has one or more so-called risk factors, which are measurable parameters that correlate with development of a disease or condition and are known in the art. An individual having one or more of these risk factors has a higher probability of developing the disease or condition than an individual without these risk factor(s).

"Nitroxyl" refers to the species HNO.

"Nitroxyl donor" or "HNO donor" refers to a compound that donates nitroxyl under physiological conditions. As used herein, nitroxyl donors may alternatively be referred to as "a compound" or "the compound." In some embodiments, the nitroxyl donor is capable of donating an effective amount of nitroxyl in vivo and has a safety profile indicating the compound would be tolerated by an individual in the amount necessary to achieve a therapeutic effect. One of ordinary skill in the art would be able to determine the safety of administering particular compounds and dosages to live subjects. One of skill in the art may also determine whether a compound is a nitroxyl donor by evaluating whether it releases HNO under physiological conditions. Compounds are easily tested for nitroxyl donation with routine experiments. Although it is impractical to directly measure whether nitroxyl is donated, several tests are accepted for determining whether a compound donates nitroxyl. For example, the compound of interest can be placed in solution, for example in phosphate buffered saline (PBS) or phosphate buffered solution at a pH of about 7.4, in a sealed container. After sufficient time for disassociation has elapsed, such as from several minutes to several hours, the headspace gas is withdrawn and analyzed to determine its composition, such as by gas chromatography and/or mass spectroscopy. If the gas N₂O is formed (which occurs by HNO dimerization), the test is positive for nitroxyl donation and the compound is a nitroxyl donor. The level of nitroxyl donating ability may be expressed as a percentage of a compound's theoretical maximum. A compound that donates a "significant level of nitroxyl" intends a compound that donates 40 % or more or 50 % or more of its theoretical maximum amount of nitroxyl. In some embodiments, the compounds for use herein donate 60 % or more of the theoretical maximum amount of nitroxyl. In some embodiments, the compounds for use herein donate 70 % or more of the theoretical maximum amount of nitroxyl. In some embodiments, the compounds for use herein donate 80% or more of the theoretical maximum amount of nitroxyl. In some embodiments, the compounds for use herein donate 90% or more of the theoretical maximum amount of nitroxyl. In some embodiments, the compounds for use herein donate between about 70% and about 90% of the theoretical maximum amount of nitroxyl. In some embodiments, the compounds for use herein donate between about 85 % and about 95 % of the theoretical maximum amount of nitroxyl. In some embodiments, the compounds for use herein donate between about 90 % and about 95 % of the theoretical maximum amount of nitroxyl. Compounds that donate less than 40% or less than 50 % of their theoretical amount of nitroxyl are still nitroxyl donors and may be used in the invention disclosed herein. A compound that donates less than 50 % of the theoretical amount of nitroxyl may be used in the methods described, and may require higher dosing levels as compared to compounds that donate a significant level of nitroxyl. Nitroxyl donation also can be detected by exposing the test compound to metmyoglobin (Mb³⁺). Nitroxyl reacts with Mb³⁺ to form an Mb²⁺-NO complex, which can be detected by changes in the ultraviolet/visible spectrum or by Electron Paramagnetic Resonance (EPR). The Mb²⁺-NO complex has an EPR signal centered around a g-value of about 2. Nitric oxide, on the other hand, reacts with Mb³⁺ to form an Mb³⁺-NO complex that is EPR silent. Accordingly, if the candidate compound reacts with Mb³⁺ to form a complex detectable by common methods, such as ultraviolet/visible or EPR, then the test is positive for nitroxyl donation. Testing for nitroxyl donation may be performed at physiologically relevant pH. Examples of nitroxyl donors include, without limitation, sodium dioxotrinitrate ("Angeli's salt" or "AS"), *N-*hydroxybenzenesulfonamide ("Piloty's acid" or "PA"), and the compounds disclosed in US Patent No. 6,936,639, US Patent Publication Nos. 2004/0038947, 2007/0299107 and 2009/0163487, and PCT Publication Nos. WO/2007/002444, WO/2005/074598 and WO/2009/137717.

"Pulmonary hypertension" or "PH" refers to a condition in which the pulmonary arterial pressure is elevated. The current haemodynamic definition of PH is a mean pulmonary arterial pressure (MPAP) at rest of greater than or equal to 25 mmHg.² Examples of PH include, but are not limited to, the conditions listed in the updated classification of PH (Table 1).³

**Table 1. Classification of Pulmonary Hypertension (PH):**

| | |
|---|---|
| 1. | Pulmonary artery hypertension (PAH) |
| ∘ | 1.1. Idiopathic PAH |
| ∘ | 1.2. Heritable |
| | ▪ 1.2.1. BMPR2 |
| | ▪ 1.2.2. ALK1, endoglin (with or without hereditary hemorrhagic |
| | telangiectasia |
| | ▪ 1.2.3. Unknown |
| ∘ | 1.3. Drug- and toxin-induced |
| ∘ | 1.4. Associated with: |
| | ▪ 1.4.1. Connective tissue diseases |
| | ▪ 1.4.2. Human immunodeficiency virus (HIV) infection |
| | ▪ 1.4.3. Portal hypertension |
| | ▪ 1.4.4. Congenital heart diseases |
| | ▪ 1.4.5. Schistosomiasis |
| ∘ | 1.5 Persistent pulmonary hypertension of the newborn |
| ∘ | 1'. Pulmonary veno-occlusive disease (PVOD) and/or pulmonary capillary |
| | hemangiomatosis (PCH) |
| 2. | Pulmonary hypertension owing to left heart disease |
| ∘ | 2.1. Systolic dysfunction |
| ∘ | 2.2. Diastolic dysfunction |
| ∘ | 2.3. Valvular disease |
| 3. | Pulmonary hypertension owing to lung disease and/or hypoxemia |
| ∘ | 3.1. Chronic obstructive pulmonary disease |
| ∘ | 3.2. Interstitial lung disease |
| ∘ | 3.3. Other pulmonary diseases with mixed restrictive and obstructive pattern |
| ∘ | 3.4. Sleep-disordered breathing |
| ∘ | 3.5. Alveolar hypoventilation disorders |
| ∘ | 3.6. Chronic exposure to high altitude |
| ∘ | 3.7. Developmental abnormalities |
| 4. | Chronic thromboembolic pulmonary hypertension (CTEPH) |
| 5. | Pulmonary hypertension with unclear multifactorial mechanisms |
| ∘ | 5.1. Hematologic disorders: myeoloproliferative disorders, splenectomy |
| ∘ | 5.2. Systemic disorders: sarcoidosis, pulmonary Langerhans cell |
| | histiocytosis: lymphangioleiomyomatosis, neurofibromatosis, vasculitis |
| ∘ | 5.3. Metabolic disorders: glycogen storage disease, Gaucher disease, thyroid |
| | disorders |
| ∘ | 5.4. Others: tumoral obstruction, fibrosing mediastinitis, chronic renal failure on dialysis |

| | |
|---|---|
| ² Badesch D. et al. Diagnosis and assessment of pulmonary arterial hypertension. J Am Coll Cardiol 2009; 54(Suppl.): S55-S66. ³ Simonneau G. et al. Updated clinical classification of pulmonary hypertension. J Am Coll Cardiol 2009; 54(1 Suppl): S43-54. | |

### Methods of Treating Pulmonary Hypertension

Some embodiments of the invention provide an HNO donor, a pharmaceutically acceptable salt thereof, or a pharmaceutical composition comprising the HNO donor or pharmaceutically acceptable salt thereof, for use in a method of treating, preventing or delaying the onset or development of pulmonary hypertension.

An individual is "in need thereof" if that individual has, is suspected of having or is at risk of having or developing pulmonary hypertension. Identifying an individual in need of such treatment can be in the judgment of a physician, clinical staff, emergency response personnel or other health care professional and can be subjective (e.g. opinion) or objective (*e*.*g*. measurable by a test or diagnostic method). In some embodiments, the individual is a mammal. In some embodiments, the individual is a human.

In some embodiments, the pulmonary hypertension is selected from the diseases and conditions listed above in Table 1. In some embodiments, the pulmonary hypertension is pulmonary arterial hypertension (PAH). In some embodiments, the pulmonary hypertension is pulmonary hypertension owing to left heart disease. In some embodiments, the left heart disease is left heart failure. In some embodiments, the left heart failure is systolic heart failure. In some embodiments, the left heart failure is diastolic heart failure. In some embodiments, the left heart failure is chronic or acutely decompensated. In some embodiments, the pulmonary hypertension is chronic thromboembolic pulmonary hypertension.

### Methods of Reducing Mean Pulmonary Arterial Pressure

Some embodiments of the invention provide an HNO donor, a pharmaceutically acceptable salt thereof, or a pharmaceutical composition comprising the HNO donor or pharmaceutically acceptable salt thereof, for use in a method of reducing mean pulmonary arterial pressure (MPAP). In some embodiments, the MPAP is reduced by up to about 50%. In some embodiments, the MPAP is reduced by up to about 25%. In some embodiments, the MPAP is reduced by up to 20%. In some embodiments, the MPAP is reduced by up to 15%. In some embodiments, the MPAP is reduced by up to 10%. In some embodiments, the MPAP is reduced by up to 5%. In some embodiments, the MPAP is reduced to about 12 to 16 mmHg. In some embodiments, the MPAP is reduced to about 15 mmHg.

### Nitroxyl (HNO) Donors

Examples of HNO donors include Angeli's salt, Piloty's acid, and the compounds disclosed in US Patent No. 6,936,639, US Patent Publication Nos. 2004/0038947, 2007/0299107 and 2009/0163487, and PCT Publication Nos. WO/2007/002444, WO/2005/074598 and WO/2009/137717.

In some embodiments, the HNO donor is a compound of formula (I) 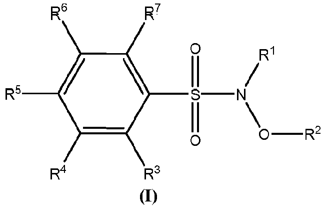 or a pharmaceutically acceptable salt thereof, wherein:
R¹ is H;
R² is H, aralkyl or heterocyclyl;
and
R³, R⁴, R⁵, R⁶ and R⁷ are independently selected from H, halo, alkylsulfonyl, *N-*hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, cycloalkoxy, cycloalkylsulfanyl, arylsulfanyl and arylsulfinyl.

In some embodiments, the HNO donor is a compound of formula (I), and:
(1) at least one of R³, R⁴, R⁵, R⁶ and R⁷ is other than H;
(2) at least one of R³, R⁴, R⁵, R⁶ and R⁷ is other than halo;
(3) when R³, R⁴, R⁶ and R⁷ are H, R⁵ is other than halo, nitro, cyano, alkyl or alkoxy;
(4) when one of R³ or R⁷ is halo and the R³ or R⁷ that is not halo is H and one of R⁴ or R⁶ is halo and the R⁴ or R⁶ that is not halo is H, R⁵ is other than halo;
(5) when R³, R⁷ and R⁵ are H and one of R⁴ and R⁶ is H, the R⁴ or R⁶ that is not H is other than *N*-hydroxysulfonamidyl, perhaloalkyl or nitro;
(6) when R⁴, R⁵ and R⁶ are H and one of R³ and R⁷ is H, the R³ or R⁷ that is not H is other than nitro or alkyl;
(7) when R³ and R⁷ are H, R⁵ is nitro and one of R⁴ and R⁶ is H, the R⁴ or R⁶ that is not H is other than halo;
(8) when R⁴ and R⁶ are nitro and R³ and R⁷ are H, R⁵ is other than dialkylamino;
(9) when R⁴ and R⁶ are H and R³ and R⁷ are alkyl, R⁵ is other than alkyl; and
(10) when R³ and R⁷ are H and R⁴ and R⁶ are nitro, R⁵ is other than dialkylamino.

In some embodiments, the HNO donor is a compound of formula (I) and:
R¹ is H;
R² is H;
R³, R⁴, R⁵, R⁶ and R⁷ are independently selected from H, halo, alkylsulfonyl, *N-*hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, cycloalkoxy, cycloalkylsulfanyl, arylsulfanyl and arylsulfinyl.

In some embodiments, the HNO donor is a compound of formula (I) and:
R¹ is H;
R² is H;
R³, R⁴, R⁵, R⁶ and R⁷ are independently selected from H, halo, alkylsulfonyl, *N-*hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, cycloalkoxy, cycloalkylsulfanyl, arylsulfanyl and arylsulfinyl, provided that:
   (1) one of R³ and R⁷ is other than H;
   (2) at least one of R³, R⁴, R⁵, R⁶ and R⁷ is other than halo;
   (3) when one of R³ or R⁷ is halo and the R³ or R⁷ that is not halo is H and one of R⁴ or R⁶ is halo and the R⁴ or R⁶ that is not halo is H, R⁵ is other than halo or hydrogen;
   (4) when R⁴, R⁵ and R⁶ are H and one of R³ and R⁷ is H, the R³ or R⁷ that is not H is other than nitro or alkyl;
   (5) when R⁴ and R⁶ are H and R³ and R⁷ are alkyl, R⁵ is other than alkyl.

In some embodiments, the HNO donor is a compound of formula (I) and R³ is halo, alkylsulfonyl, perhaloalkyl, lower alkyl, nitro or cyano.

In some embodiments, the HNO donor is a compound of formula (I); R³ is halo, alkylsulfonyl, perhaloalkyl, lower alkyl, nitro or cyano; and at least three of R⁴, R⁵, R⁶ and R⁷ are H.

In some embodiments, the HNO donor is a compound of formula (I); R³ is halo, alkylsulfonyl, perhaloalkyl, lower alkyl, nitro or cyano; and R⁴, R⁵, R⁶ and R⁷ are H.

In some embodiments, the HNO donor is a compound of formula (I) and R³ is halo, methylsulfonyl, perfluoromethyl, perfluoromethoxy, isopropyl, nitro or cyano.

In some embodiments, the HNO donor is a compound of formula (I); R³ is halo, methylsulfonyl, perfluoromethyl, perfluoromethoxy, isopropyl, nitro or cyano; and at least three of R⁴, R⁵, R⁶ and R⁷ are H.

In some embodiments, the HNO donor is a compound of formula (I); R³ is halo, methylsulfonyl, perfluoromethyl, perfluoromethoxy, isopropyl, nitro or cyano; and R⁴, R⁵, R⁶ and R⁷ are H.

Representative compounds of formula (I) include, but are not limited to, the compounds listed in Table 2.

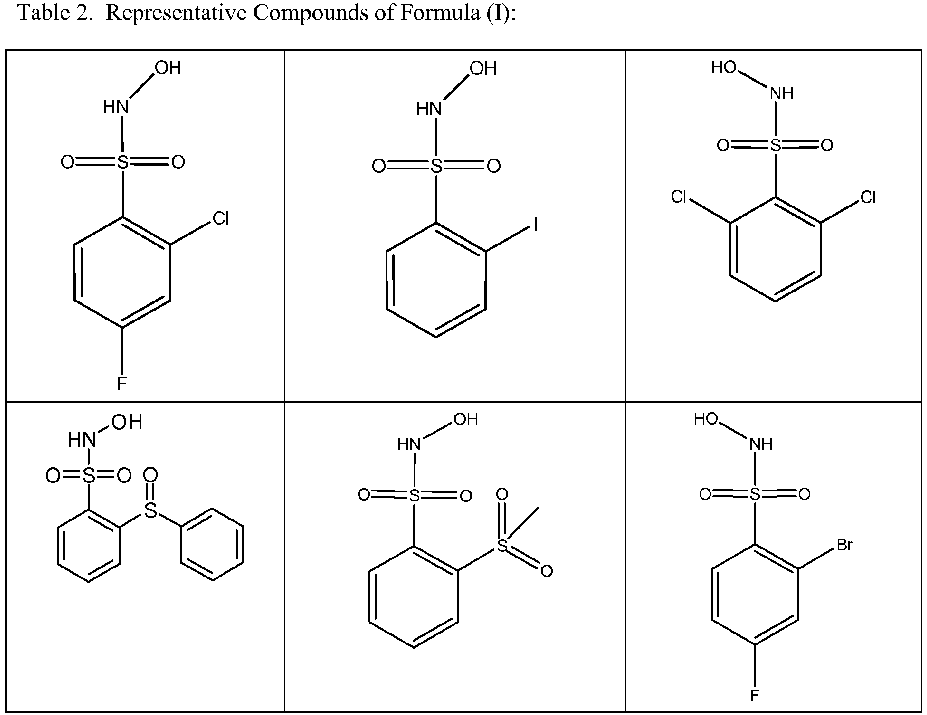

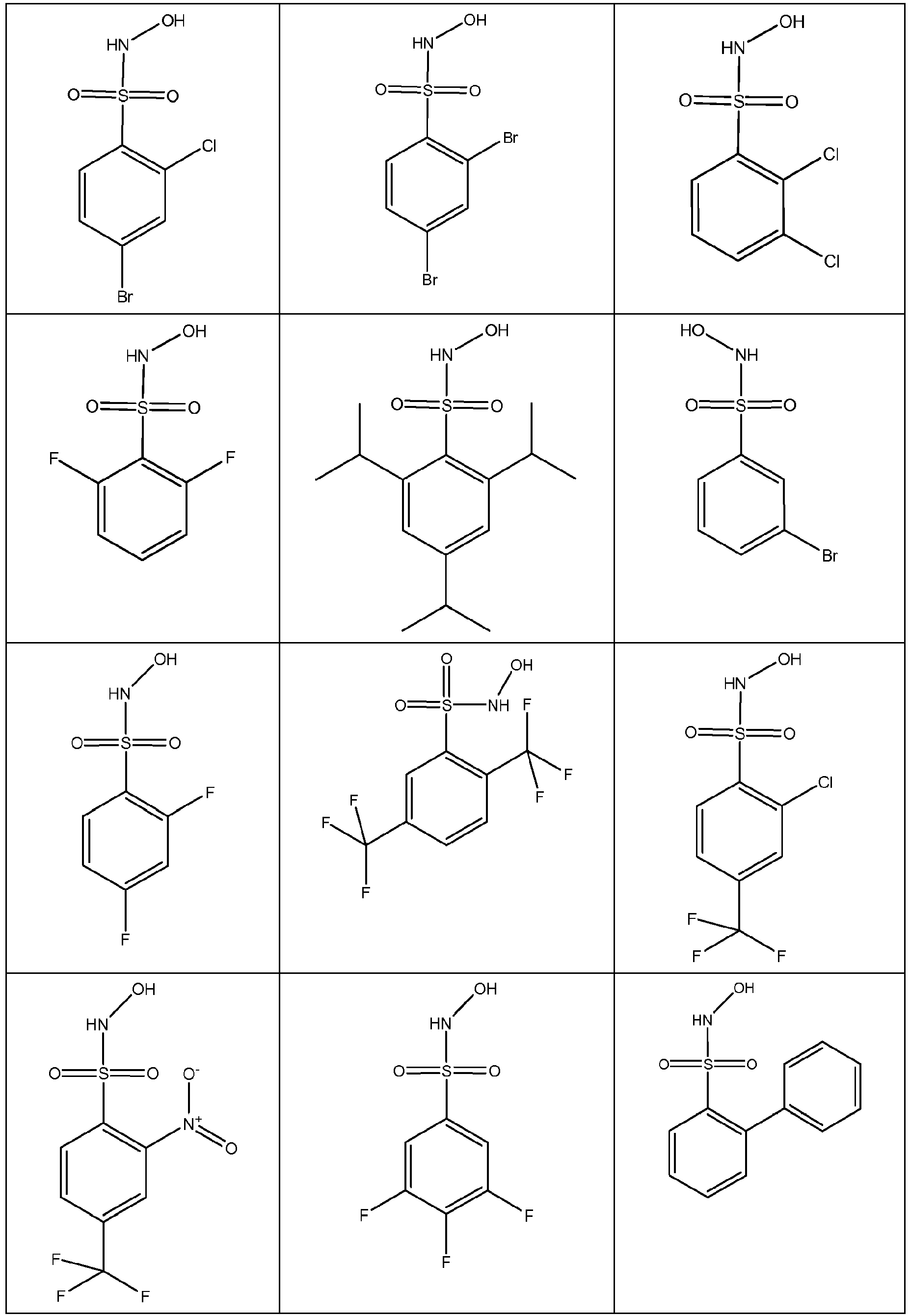

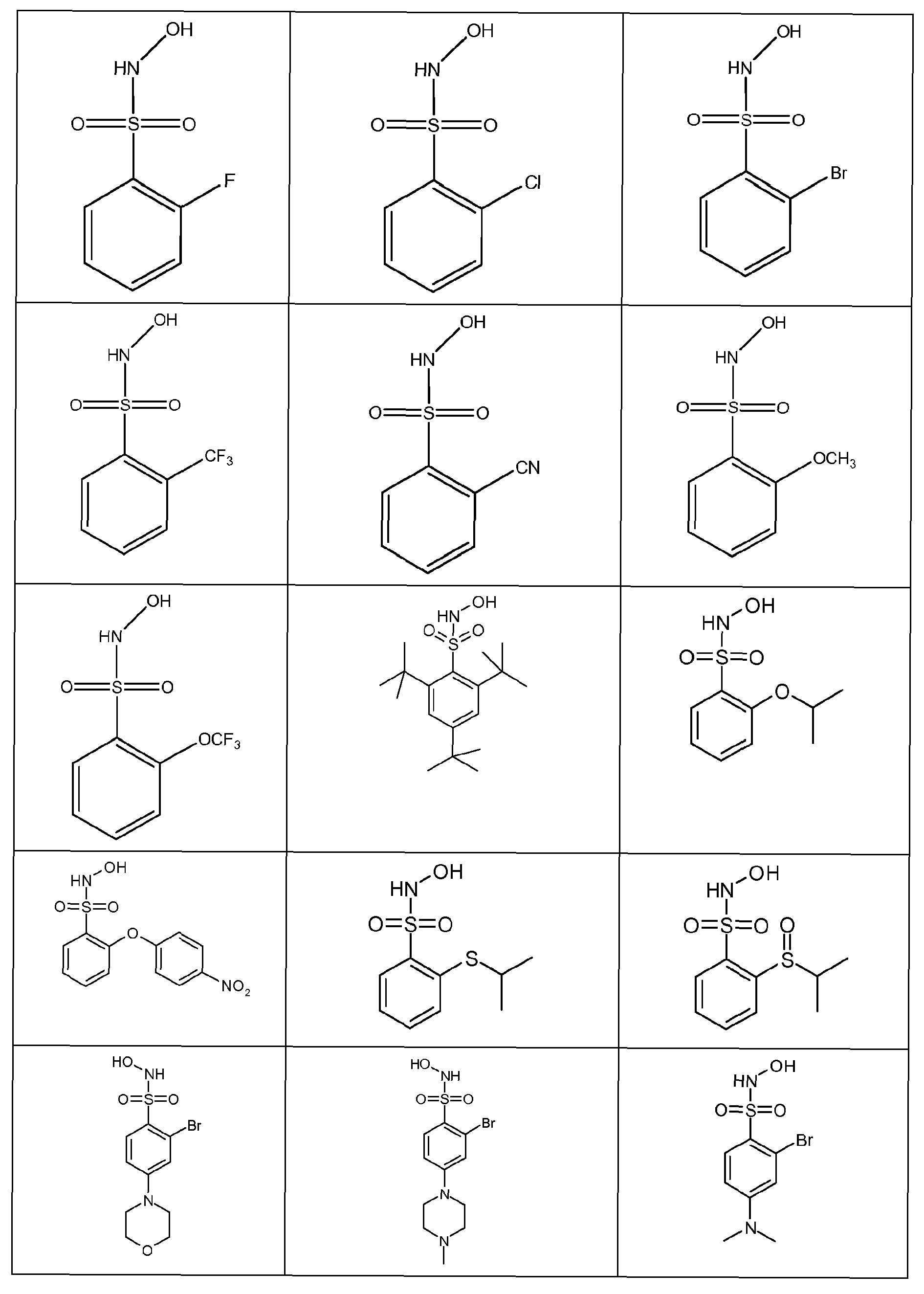

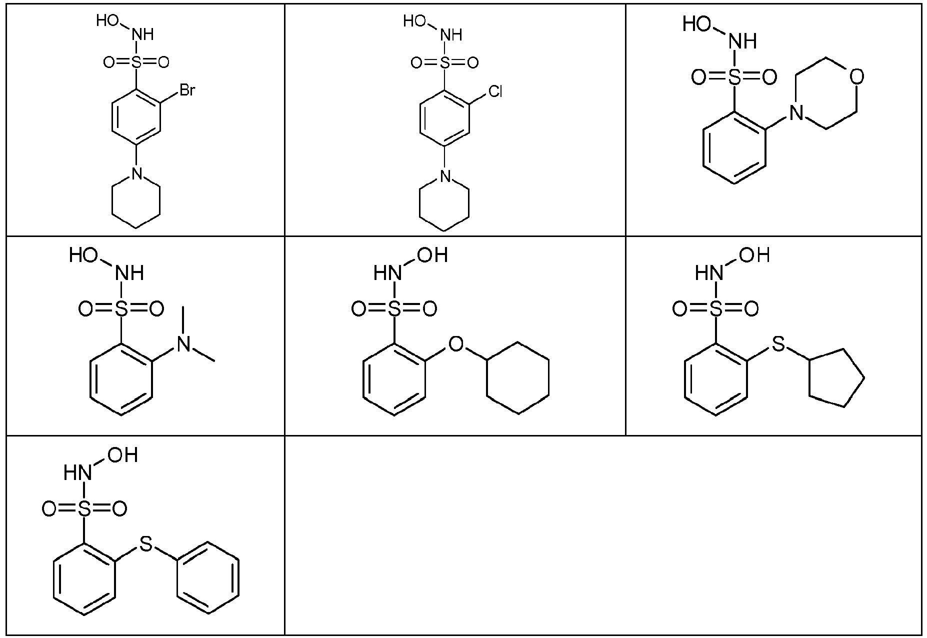

In some embodiments, the HNO donor is selected from:
2,6-Dichloro-*N*-hydroxy benzene sulfonamide;
2-Bromo-4-fluoro-*N*-hydroxy benzene sulfonamide;
2,5-Di-trifluoromethyl-*N*-hydroxy benzene sulfonamide;
2-Chloro-4-fluoro-*N-*hydroxy benzene sulfonamide;
2,3-Dichloro-*N*-hydroxy benzene sulfonamide;
2-Chloro-4-bromo-*N*-hydroxy benzene sulfonamide;
2-Nitro-4-trifluoromethyl-*N*-hydroxy benzene sulfonamide;
2-Iodo-*N*-hydroxy benzene sulfonamide;
*N*-Hydroxy-2-methanesulfonyl benzene sulfonamide;
2,4-Di-bromo-*N-*hydroxy benzene sulfonamide;
2-Chloro-4-trifluoromethyl-*N-*hydroxy benzene sulfonamide;
2,4,6-Tri-isopropyl-*N-*hydroxy benzene sulfonamide;
2,4-Di-fluoro-*N-*hydroxy benzene sulfonamide;
2-Fluoro-*N-*hydroxy benzene sulfonamide;
2-Bromo-*N-*hydroxy benzene sulfonamide;
2-(Trifluoromethyl)-*N-*hydroxy benzenesulfonamide;
*N-*Hydroxy-2-phenyl benzene sulfonamide; and
pharmaceutically acceptable salts thereof.

In some embodiments, the HNO donor is 2-Iodo-*N-*hydroxy benzene sulfonamide.

In some embodiments, the HNO donor is *N*-Hydroxy-2-methanesulfonyl benzene sulfonamide.

In some embodiments, the HNO donor is 2-Fluoro-*N-*hydroxybenzenesulfonamide.

In some embodiments, the HNO donor is 2-Chloro-*N-*hydroxybenzenesulfonamide.

In some embodiments, the HNO donor is 2-Bromo-*N-*hydroxybenzenesulfonamide.

In some embodiments, the HNO donor is 2-(Trifluoromethyl)-*N-*hydroxybenzenesulfonamide.

In some embodiments, the HNO donor is a compound of formula (II) 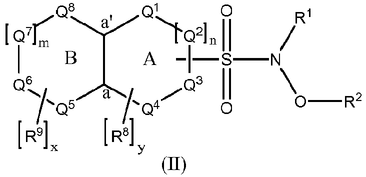 or a pharmaceutically acceptable salt thereof, wherein:
R¹ is H;
R² is H, aralkyl or heterocyclyl;
m and n are independently an integer from 0 to 1;
x is an integer from 0 to 4; y is an integer from 0 to 3;
A is a cycloalkyl, heterocycloalkyl, aromatic or heteroaromatic ring containing ring moieties Q¹, Q², Q³ and Q⁴, which are taken together with the carbons at positions a and a' to form ring A; B is a cycloalkyl, heterocycloalkyl, aromatic or heteroaromatic ring containing ring moieties Q⁵, Q⁶, Q⁷ and Q⁸, which are taken together with the carbons at positions a and a' to form ring B; Q¹, Q², Q³, Q⁴, Q⁵, Q⁶, Q⁷and Q⁸ are independently selected from C, CH₂, CH, N, NR¹⁰, O and S.

In some embodiments, the HNO donor is a compound of formula (II); R⁸ and R⁹ is independently selected from Cl, F, I, Br, SO₂CH₃, SO₂NHOH, CF₃, CH₃, NO₂, phenyl, CN, OCH₃, OCF₃, *t*-Bu, O-*i*Pr, 4-nitrophenyloxy (OPh4-NO₂), propane-2-thiyl (SCH(CH₃)₂), propane-2-sulfinyl (S(O)CH(CH₃)₂), morpholino, *N-*methyl-piperazino, dimethylamino, piperidino, cyclohexyloxy, cyclopentylsulfanyl, phenylsulfanyl and phenylsulfinyl; and R¹⁰ is H, alkyl, acyl or sulfonyl, provided that when rings A and B form naphthalene, x is an integer from 1 to 3 or y is an integer from 2 to 3.

Included in any of the embodiments of formula (II) described above are the following additional embodiments. In some embodiments, R¹ is H; and R² is H, benzyl or tetrahydropyran-2-yl. In some embodiments, A and B form a benzofuran, benzothiophene, benzoimidazole, *N*-alkylbenzoimidazole (such as *N*-methylbenzoimidazole), *N-*acylbenzoimidazole (such as N-C(O)CH₃benzoimidazole), benzothiazole or benzooxazole. In some embodiments, A and B form a benzofuran. In some embodiments, A and B form a benzofuran; and x and y are 0. In some embodiments, A and B form a benzothiophene. In some embodiments, A and B form a benzothiophene; y is 0; and x is 1. In some embodiments, A and B form naphthyl; x is 0; y is 1; and R⁸ is halo. In some embodiments, ring A is phenyl; and ring B is a heteroaryl group, such as when rings A and B form quinoline and ring B is the nitrogen containing ring. In some embodiments, y is 0; x is 1; and R⁹ is halo, alkyl or perhaloalkyl. In some embodiments, y is 0; and x is 2.

Representative compounds of formula (II) include, but are not limited to, the compounds listed in Table 3.

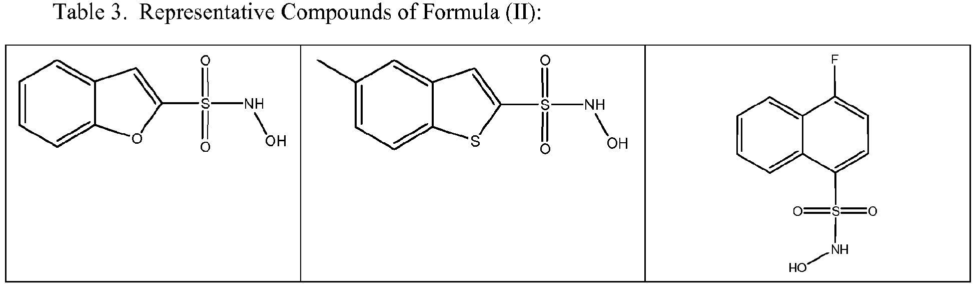

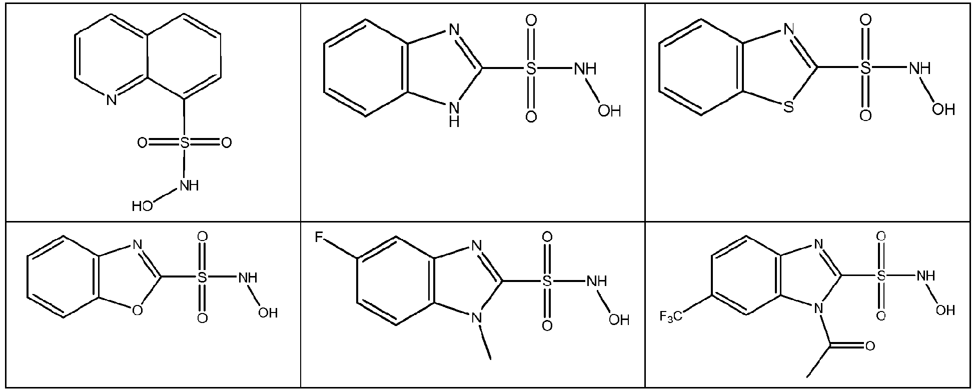

In some embodiments, the HNO donor is a compound of formula (III) 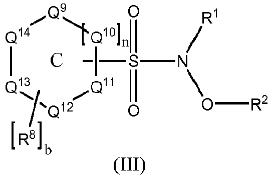 or a pharmaceutically acceptable salt thereof, wherein:
R¹ is H;
R² is H, aralkyl or heterocyclyl;
n is an integer from 0 to 1;
b is an integer from 0 to 4;
C is a heteroaromatic ring containing ring moieties Q⁹, Q¹⁰, Q¹¹, Q¹², Q¹³and Q¹⁴ that are independently selected from C, CH₂, CH, N, NR¹⁰ O and S, provided that at least one of Q⁹, Q¹⁰, Q¹¹, Q¹², Q¹³and Q¹⁴ is N, NR¹⁰, O or S;
each R⁸ is independently selected from halo, alkylsulfonyl, *N*-hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, NH₂, OH, C(O)OH, C(O)Oalkyl, NHC(O)alkylC(O)OH, C(O)NH₂, NHC(O)alkylC(O)alkyl, NHC(O)alkenylC(O)OH, NHC(O)NH₂, OalkylC(O)Oalkyl, NHC(O)alkyl, C(=N-OH)NH₂, cycloalkoxy, cycloalkylsulfanyl, arylsulfanyl, and arylsulfinyl; and
R¹⁰ is H, alkyl, acyl or sulfonyl.

In some embodiments, the HNO donor is a compound of formula (III); and each R² is H.

In some embodiments, the HNO donor is a compound of formula (III); and each R⁸ is independently selected from Cl, F, I, Br, SO₂CH₃ SO₂NHOH, CF₃, CH₃, NO₂, phenyl, CN, OCH₃, OCF₃, *t*-Bu, O-*i*Pr, 4-nitrophenyloxy (OPh4-NO₂), propane-2-thiyl (SCH(CH₃)₂), propane-2-sulfinyl (S(O)CH(CH₃)₂), morpholino, *N*-methyl-piperazino, dimethylamino, piperidino, cyclohexyloxy, cyclopentylsulfanyl, phenylsulfanyl and phenylsulfinyl.

In some embodiments, the HNO donor is a compound of formula (III); and each R⁸ is independently selected from F, Br, Cl, CF₃, phenyl, methyl, -SO₂NHOH, morpholino, piperidino, and 4-methyl-piperazino.

Included in any of the embodiments of formula (III) described above are the following additional embodiments. In some embodiments, the HNO donor is a compound of formula (III); R¹ is H; and R² is H, benzyl or tetrahydropyran-2-yl. In some embodiments, n is 0; and C is a thiophene, isoxazole, pyrazole, pyrrole, imidazole, furan, thiazole, triazole, *N-*methylimidazole or thiadiazole. In some embodiments, n is 0; and C is a thiophene, isoxazole, pyrazole, pyrrole, imidazole, furan, thiazole, triazole, *N*-methylimidazole or thiadiazole; and either (1) b is 1 and R⁸ is either a halo (such as Cl or Br), nitro, alkyl (such as methyl), cyano or (2) b is 2 and each R⁸ is independently a halo. In some embodiments, n is 1; and C is a pyrimidine, pyrazine or pyridine. In some embodiments, n is 1; C is a pyrimidine, pyrazine or pyridine; b is either 0 or 1; and R⁸ is halo or heterocyclyl if b is 1. In some embodiments, n is 1; and C is a pyrimidine, pyrazine or pyridine; b is 1; and R⁸ is chloro, morpholino, piperidino or *N-*methylpiperizino. In some embodiments, C is thiophene; and b is 1. In some embodiments, C is thiophene; b is 1; and R⁸ is halo. In some embodiments, C is thiophene and b is 0. In some embodiments, C is thiophene; b is 1; R⁸ is F, Br, Cl. CF₃, phenyl, methyl, -SO₂CH₃, -SO₂NHOH, morpholino, piperidino, and 4-methyl-piperazino.

Representative compounds of formula (III) include, but are not limited to, the compounds listed in Table 4.

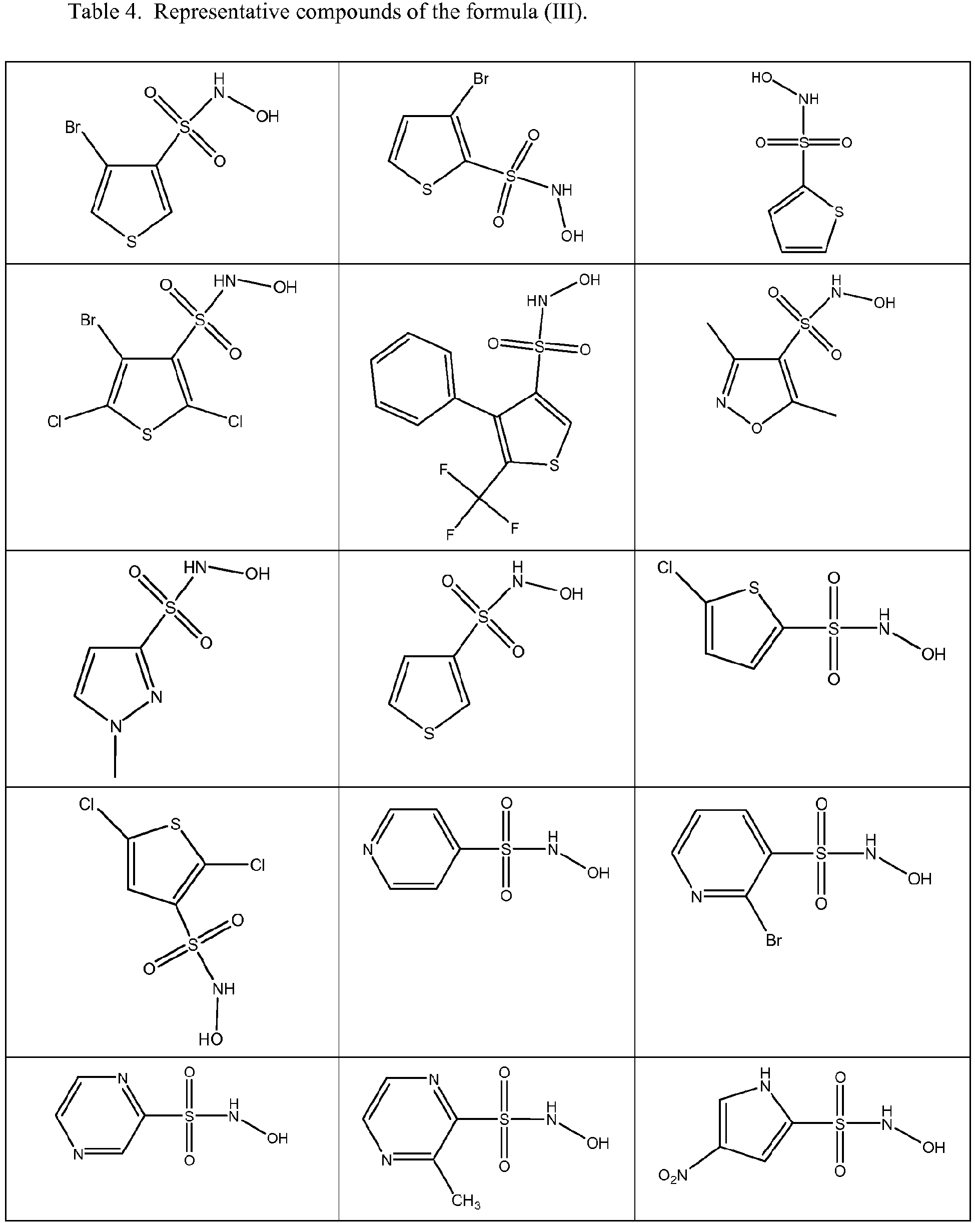

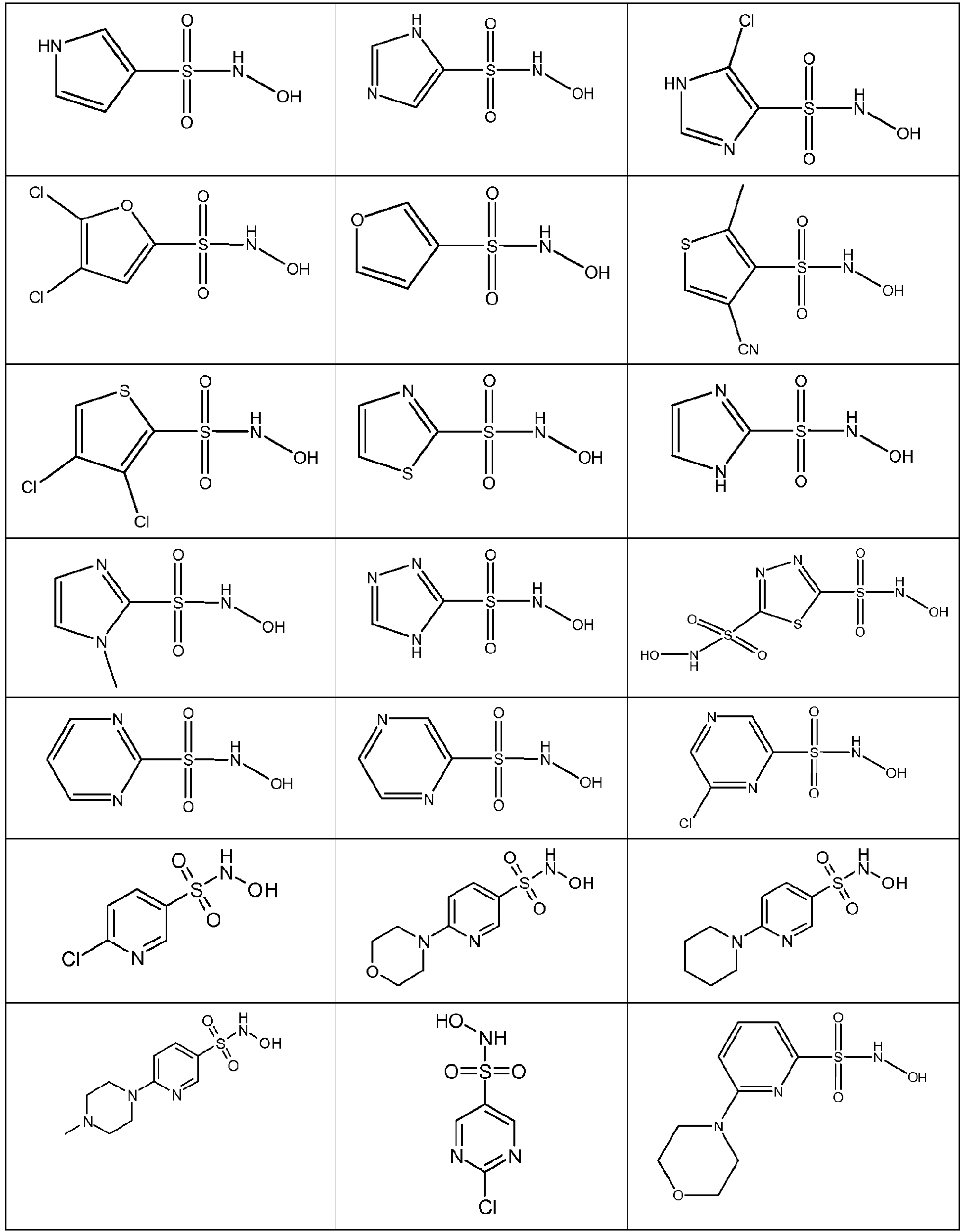

In some embodiments, the HNO donor is a compound of formula (IV) 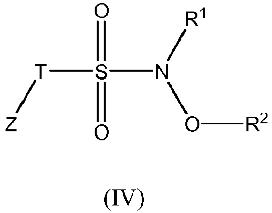 or a pharmaceutically acceptable salt thereof, wherein:
R¹ is H;
R² is H, aralkyl or heterocyclyl;
T is alkyl or substituted alkyl (which includes a cycloalkyl or substituted cycloalkyl); and
Z is an electron withdrawing group.

In some embodiments, T is C₁ to C₆ branched alkyl, such as isopropyl, t-butyl or sec-butyl. In some embodiments, T is C₁ to C₆ branched alkyl, such as isopropyl, t-butyl or sec-butyl; and Z is F, Cl, Br, I, -CN, -CF₃, -NO₂, -SH, -C(O)H, -C(O)alkyl, -C(O)Oalkyl, - C(O)OH, -C(O)Cl, -S(O)₂OH, -S(O)₂NHOH or -NH₃.

Included in any of the embodiments of formula (IV) described above are the following embodiments. In some embodiments, R¹ is H; and R² is H, benzyl or tetrahydropyran-2-yl.

Representative compounds of formula (IV) include, but are not limited to, the compounds listed in Table 5.

### Table 5. Representative compounds of formula (IV).

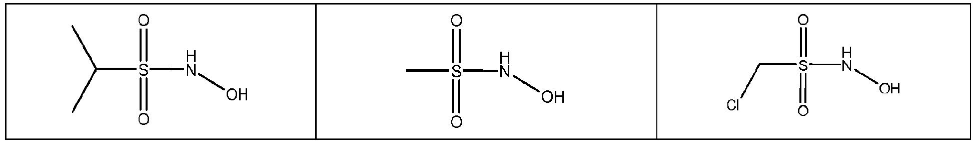

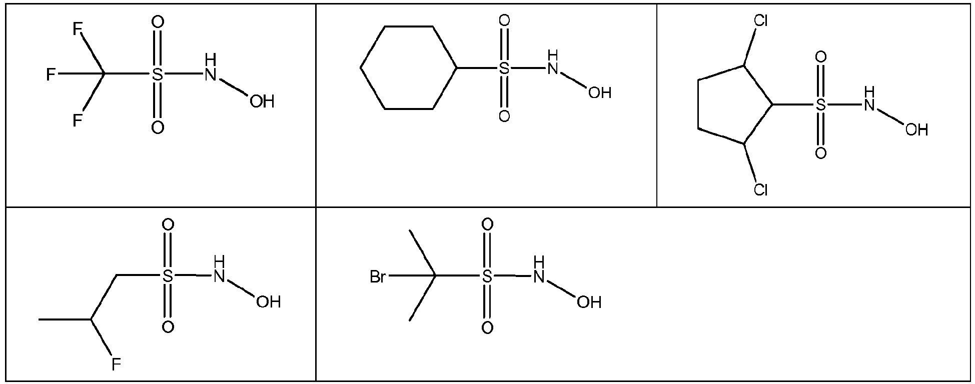

In some embodiments, the HNO donor is a compound of the formula (Ia): 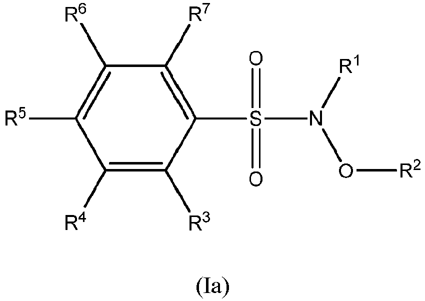 or a pharmaceutically acceptable salt thereof, wherein:
R¹ is H;
R² is H, aralkyl or heterocyclyl;
R³, R⁴, R⁵, R⁶ and R⁷ are independently selected from H, halo, alkylsulfonyl, *N-*hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, cycloalkoxy, cycloalkylsulfanyl, arylsulfanyl, arylsulfinyl, carboxyl, carboxyl ester, acylamino and sulfonylamino, provided that at least one of R³, R⁴, R⁵, R⁶ and R⁷ is carboxyl, carboxyl ester, acylamino or sulfonylamino.

In some embodiments, the HNO donor is a compound of formula (Ia), wherein:
R¹ is H;
R² is H, aralkyl or heterocyclyl;
R⁴, R⁵ and R⁶ are independently H, halo, alkylsulfonyl, *N*-hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, cycloalkoxy, cycloalkylsulfanyl, arylsulfanyl, arylsulfinyl, carboxyl, carboxyl ester, acylamino or sulfonylamino;
at least one of R³ and R⁷ is an electron withdrawing group or a group that sterically hinders the sulfonyl moiety;
provided that at least one of R³, R⁴, R⁵, R⁶ and R⁷ is carboxyl, carboxyl ester, acylamino or sulfonylamino.

In some embodiments, the HNO donor is a compound of formula (Ia), wherein at least one of R³ and R⁷ is an electron withdrawing group.

In some embodiments, the HNO donor is a compound of formula (Ia), wherein both R³ and R⁷ are electron withdrawing groups.

In some embodiments, the HNO donor is a compound of formula (Ia), wherein at least one of R³ and R⁷ is a group that sterically hinders the sulfonyl moiety of compound (Ia).

In some embodiments, the HNO donor is a compound of formula (Ia), wherein at least one of R³ and R⁷ is a branched alkyl group, such as *i*-propyl or *t*-butyl.

In some embodiments, the HNO donor is a compound of formula (Ia), wherein both R³ and R⁷ are alkyl groups, provided that at least one of the alkyl groups is a branched alkyl group, such as when both groups are isopropyl or when one group is ethyl and the other is *sec*-butyl.

In some embodiments, the HNO donor is a compound of formula (Ia), wherein:
one of R³ and R⁷ is an electron withdrawing group; and
the R³ or R⁷ that is not an electron withdrawing group is an alkyl group, which may be a branched alkyl group such as isopropyl.

In some embodiments, the HNO donor is a compound of formula (Ia), wherein:
R¹ is H; R² is H, benzyl or tetrahydropyran-2-yl;
R³, R⁴, R⁵, R⁶ and R⁷ are independently selected from H, Cl, F, I, Br, SO₂CH₃ SO₂NHOH, CF₃, NO₂, phenyl, CN, OCH₃, OCF₃, *t*-Bu, O-*i*Pr, 4-nitrophenyloxy (OPh4-NO₂), propane-2-thiyl (SCH(CH₃)₂), propane-2-sulfinyl (S(O)CH(CH₃)₂), morpholino, *N-*methyl-piperazino, dimethylamino, piperidino, cyclohexyloxy, cyclopentylsulfanyl, phenylsulfanyl, phenylsulfinyl, carboxyl, carboxyl ester, acylamino and sulfonylamino, provided that at least one of R³, R⁴, R⁵, R⁶ and R⁷ is carboxyl, carboxyl ester, acylamino or sulfonylamino.

Included in any of the embodiments of formula (Ia) described above are the following additional embodiments.

In some embodiments, R¹ is H and R² is H, benzyl or tetrahydropyran-2-yl. In some embodiments, at least two of R³, R⁴, R⁵, R⁶ and R⁷ are halo, such as the compound of formula (I) wherein R⁵ is halo (such as F or Br) and one of R³ and R⁷ is halo (such as Br or Cl), or wherein both R³ and R⁷ or both R³ and R⁴ are halo (such as when both are Cl or both are F or one is Cl and one is F), and the remaining substituents are as described in the embodiments above. In some embodiments, at least one of R³, R⁴, R⁵, R⁶ and R⁷ is -S(O)₂alkyl, such as when R³ or R⁷ is -S(O)₂CH₃. In some embodiments, at least one of R³, R⁵ and R⁷ is a perhaloalkyl, such as when R³ is CF₃ or when R³ and R⁵ are CF₃. In some embodiments, R⁵ is CF₃, and at least one of R³ and R⁷ is other than H, such as when R⁵ is CF₃ and R³ is NO₂ or Cl. In some embodiments, at least one of R³, R⁴, R⁵, R⁶ and R⁷ is aryl, such as when at least one of R³ and R⁷ is aryl, such as phenyl. In some embodiments, at least one of R³, R⁴, R⁵, R⁶ and R⁷ is heterocyclyl, such as when at least one of R³, R⁵ and R⁷ is heterocyclyl or substituted heterocylcyl, such as morpholino, *N*-methyl, piperizino and piperidino. In some embodiments, at least one of R³, R⁴, R⁵, R⁶ and R⁷ is cycloalkoxy or cycloalkylsulfanyl, such as when at least one of R³, R⁵ and R⁷ is cyclohexyloxy, cyclopentyloxy, cyclohexylsulfanyl or cyclopentylsulfanyl. In some embodiments, at least one of R³, R⁴, R⁵, R⁶ and R⁷ is arylsulfanyl or arylsulfinyl, such as when at least one of R³, R⁵ and R⁷ is phenylsulfanyl or phenylsulfinyl.

In some embodiments, at least one of R³, R⁴, R⁵, R⁶ and R⁷ is carboxyl. In some embodiments, R⁴ is carboxyl, R³, R⁵ and R⁶ are H, and R⁷ is H or halo. In some embodiments, R⁴ is carboxyl, R³, R⁵ and R⁶ are H, R⁷ is H or halo and R¹ and R² are H. In some embodiments, at least one of R³, R⁴, R⁵, R⁶ and R⁷ is -COO-alkyl. In some embodiments, R³ is -COO-alkyl and R⁴, R⁵, R⁶ and R⁷ are H. In some embodiments, R³ is - COO-alkyl, R⁴, R⁵, R⁶ and R⁷ are H and R¹ and R² are H. In some embodiments, R⁴ is - COO-alkyl, one of R⁶ and R⁷ is -SR¹¹, aryl, -OR¹¹, nitro, cyano, acyl, -S(O)₂NHO sulfonylamino, C₁-C₂perfluoroalkyl, lower alkyl or amino, and the R⁶ or R⁷ that is not SR¹¹, aryl, -OR¹¹, nitro, cyano, acyl, -S(O)₂NHOH, sulfonylamino, C₁-C₂perfluoroalkyl, lower alkyl or amino is hydrogen. In some embodiments, R⁴ is -COO-alkyl, one of R⁶ and R⁷ is - SR¹¹, aryl, -OR¹¹, nitro, cyano, acyl, -S(O)₂NHOH, sulfonylamino, C₁-C₂perfluoroalkyl, lower alkyl or amino, the R⁶ or R⁷ that is not -SR¹¹, aryl, -OR¹¹, nitro, cyano, acyl, - S(O)₂NHOH, sulfonamino, C₁-C₂perfluoroalkyl, lower alkyl or amino is hydrogen, and R¹, R², R³ and R⁵ are hydrogen.

In some embodiments, at least one of R³, R⁴, R⁵, R⁶ and R⁷ is -COO-substituted alkyl. In some embodiments, R⁴ is -COO-substituted alkyl, R³, R⁵ and R⁶ are H, and R⁷ is halo. In some embodiments, R⁴ is -COO-substituted alkyl, R⁷ is halo, R³, R⁵ and R⁶ are H, and R¹ and R² are H.

In some embodiments, at least one of R³, R⁴, R⁵, R⁶ and R⁷ is -C(O)NH₂. In some embodiments, at least one of R³, R⁴, R⁵, R⁶ and R⁷ is -C(O)NRₐR_{b}, wherein Rₐ is hydrogen and R_{b} is alkyl. In some embodiments, R⁴ is -C(O)NRₐR_{b}, wherein Rₐ is hydrogen, R_{b} is lower alkyl, R³, R⁵ and R⁶ are H, and R⁷ is halo. In some embodiments, R⁴ is -C(O)NRₐR_{b}, wherein Rₐ is hydrogen, R_{b} is lower alkyl, R³, R⁵ and R⁶ are H, R⁷ is halo, and R¹ and R² are H. In some embodiments, R_{b} is a C₂-C₄ alkyl, such as ethyl, propyl or butyl. In some embodiments, R_{b} is a branched lower alkyl, e.g., isopropyl or isobutyl.

In some embodiments, at least one of R³, R⁴, R⁵, R⁶ and R⁷ is -C(O)NRₐR_{b} wherein Rₐ is alkyl, substituted alkyl or hydrogen, and R_{b} is substituted alkyl. In some embodiments, R⁴ is -C(O)NRₐR_{b} wherein Rₐ is alkyl, substituted alkyl or hydrogen, R_{b} is substituted alkyl, R³, R⁵ and R⁶ are H, and R⁷ is halo. In some embodiments, R⁴ is -C(O)NRₐR_{b} wherein Rₐ is alkyl, substituted alkyl or hydrogen, R_{b} is substituted alkyl, R³, R⁵ and R⁶ are H, R⁷ is halo, and R¹ and R² are H. In some embodiments, R⁴ is -C(O)NRₐR_{b} wherein Rₐ is lower alkyl, substituted lower alkyl or hydrogen, R_{b} is substituted lower alkyl, R³, R⁵ and R⁶ are H, and R⁷ is halo. In some embodiments, when R_{b} is a substituted lower alkyl group, it is a lower alkyl substituted with hydroxyl, carboxyl, amino or alkoxy. For example, the invention embraces compounds wherein R⁴ is -C(O)NRₐR_{b} wherein Rₐ is hydrogen, methyl, ethyl, or a lower alkyl substituted with hydroxyl or alkoxy, R_{b} is a lower alkyl substituted with hydroxyl, carboxyl, amino or alkoxy, R³, R⁵ and R⁶ are H, and R⁷ is halo; in further embodiments, R¹ and R² are H. In some embodiments, at least one of R³, R⁴, R⁵, R⁶ and R⁷ is -C(O)NRₐR_{b} wherein Rₐ and R_{b} are independently alkyl. In some embodiments, R⁴ is -C(O)NRₐR_{b} wherein Rₐ and R_{b} are independently alkyl, R³, R⁵ and R⁶ are H, and R⁷ is halo. In some embodiments, R⁴ is -C(O)NRₐR_{b} wherein Rₐ and R_{b} are independently alkyl, R³ and R⁵ are hydrogen and one of R⁶ and R⁷ is -SR¹¹, aryl, -OR¹¹, nitro, cyano, acyl, -S(O)₂NHOH, sulfonamino, C₁-C₂perfluoroalkyl, lower alkyl or amino, and the R⁶ or R⁷ that is not -SR¹¹, aryl, -OR¹¹, nitro, cyano, acyl, -S(O)₂NHOH, sulfonamino, C₁-C₂perfluoroalkyl, lower alkyl or amino is hydrogen. In some embodiments, R⁴ is -C(O)NRₐR_{b} wherein Rₐ and R_{b} are independently alkyl, R³, R⁵ and R⁶ are H, R⁷ is halo, and R¹ and R² are H. Rₐ and R_{b} may be the same or different, e.g., Rₐ and R_{b} may be both methyl or ethyl, or one is methyl and the other is ethyl. In some embodiments, at least one of R³, R⁴, R⁵, R⁶ and R⁷ is -C(O)NRₐR_{b} wherein Rₐ and R_{b} are taken together with the nitrogen to which they are attached to form a heterocyclic or substituted heterocyclic ring. In some embodiments, R⁴ is -C(O)NRₐR_{b} wherein Rₐ and R_{b} are taken together with the nitrogen to which they are attached to form a heterocyclic or substituted heterocyclic ring. In some embodiments, R⁴ is -C(O)NRₐR_{b} wherein Rₐ and R_{b} are taken together with the nitrogen to which they are attached to form a heterocyclic or substituted heterocyclic ring, R³, R⁵ and R⁶ are H and R⁷ is halo. In some embodiments, R⁴ is -C(O)NRₐR_{b} wherein Rₐ and R_{b} are taken together with the nitrogen to which they are attached to form a heterocyclic or substituted heterocyclic ring, R³, R⁵ and R⁶ are H, R⁷ is halo, and R¹ and R² are H. In some embodiments, Rₐ and R_{b} are taken together with the nitrogen to which they are attached to form a heterocyclic ring, such as morpholino. In some embodiments, R⁴ is -C(O)NRₐR_{b}, and Rₐ and R_{b} are taken together with the nitrogen to which they are attached to form a heterocyclic ring selected from piperazinyl, azetidinyl, pyrrolidinyl, piperidinyl, thiomorpholinyl and morpholinyl. In some embodiments, R⁴ is -C(O)NRₐR_{b}, and Rₐ and R_{b} are taken together with the nitrogen to which they are attached to form a heterocyclic ring substituted with 1 or 2 moieties selected from lower alkyl, carboxylester, acyl, halo, amino, hydroxyl, substituted lower alkyl, oxo and alkoxy. For example, in embodiments wherein R⁴ is -C(O)NRₐR_{b}, Rₐ and R_{b} are taken together with the nitrogen to which they are attached to form a substituted heterocyclic ring selected from 2,6-dimethylpiperaz-4-yl, 1-isopropylpiperaz-4-yl, 1-(piperazin-4-yl)ethanone, tert-butyl piperaz-4-yl-l-carboxylate, 4-fluoropiperidyl, 4,4-difluoropiperidyl, 4-aminopiperidyl, 4-hydroxypiperidyl, 4-oxopiperidinyl, 4-methoxypiperidyl, 4-(2-hydroxyethyl)piperidyl, 2-(piperid-4-yl)-ethoxyethanol, 3-hydroxy-azetidinyl, 2-oxo-piperazin-4-yl and 1-methyl-2-oxo-piperazin-4-yl.

In some embodiments, at least one of R³, R⁴, R⁵, R⁶ and R⁷ is -SO₂NH₂.

In some embodiments, at least one of R³, R⁴, R⁵, R⁶ and R⁷ is -SO₂NR-alkyl wherein R is hydrogen. In some embodiments, at least one of R³, R⁴, R⁵, R⁶ and R⁷ is -SO₂NR-alkyl wherein R is alkyl. In some embodiments, R⁴ is -SO₂NR-alkyl wherein R is alkyl and R³, R⁵, R⁶ and R⁷ are hydrogen. For example, in some embodiments, R⁴ is -SO₂N(lower alkyl)₂ and R³, R⁵, R⁶ and R⁷ are hydrogen, wherein the lower alkyl substituents may be the same or different, e.g., R⁴ may be -SO₂N(Et)₂ or -SO₂N(Et)(Me).

In some embodiments, at least one of R³, R⁴, R⁵, R⁶ and R⁷ is -SO₂NR₂, wherein the two R groups are taken together with the nitrogen to which they are attached to form a heterocyclic or substituted heterocyclic ring. In some embodiments, R³ is -SO₂NR₂, wherein the two R groups are taken together with the nitrogen to which they are attached to form a heterocyclic or substituted heterocyclic ring. In some embodiments, R³ is -SO₂NR₂, wherein the two R groups are taken together with the nitrogen to which they are attached to form a heterocyclic or substituted heterocyclic ring, and R⁴, R⁵, R⁶ and R⁷ are H. In some embodiments, R³ is -SO₂NR₂, wherein the two R groups are taken together with the nitrogen to which they are attached to form a heterocyclic or substituted heterocyclic ring, R⁴, R⁵, R⁶ and R⁷ are H, and R¹ and R² are H. In some embodiments, at least one of R³, R⁴, R⁵, R⁶ and R⁷ is -SO₂NR₂, wherein the two R groups are taken together with the nitrogen to which they are attached to form a heterocyclic ring, such as a morpholino ring.

Representative compounds of the formula (Ia) include, but are not limited to, the compounds listed in Table 6.

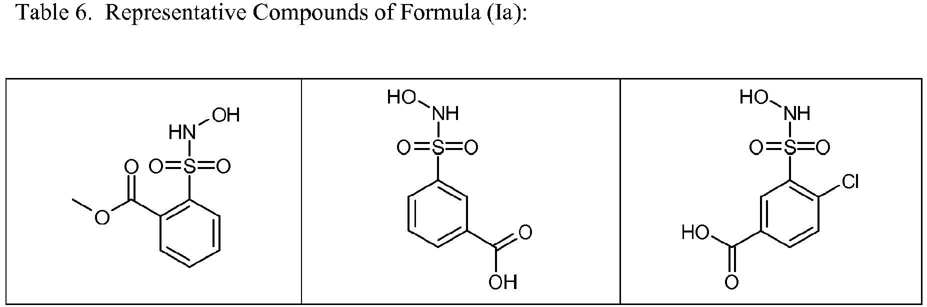

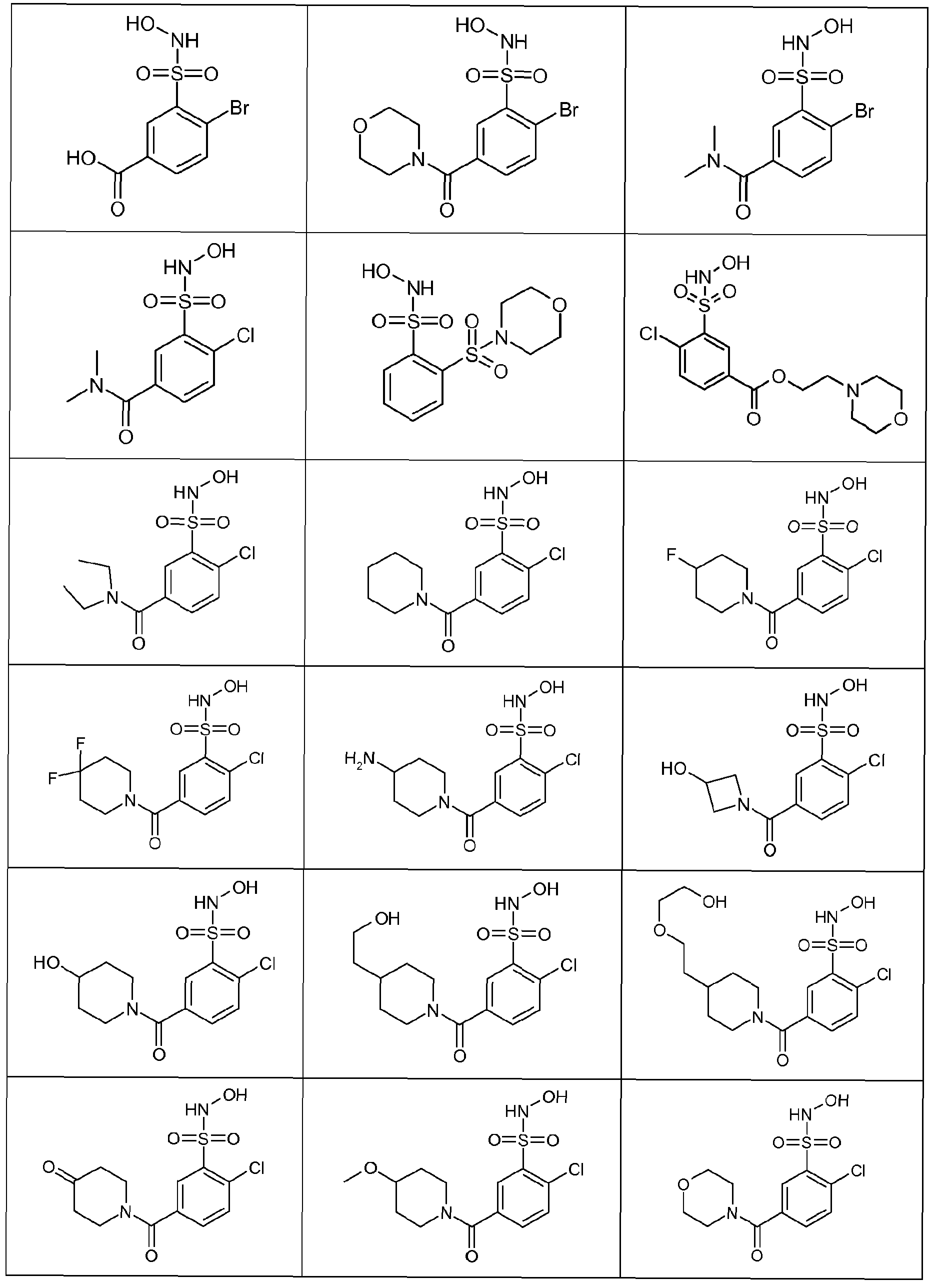

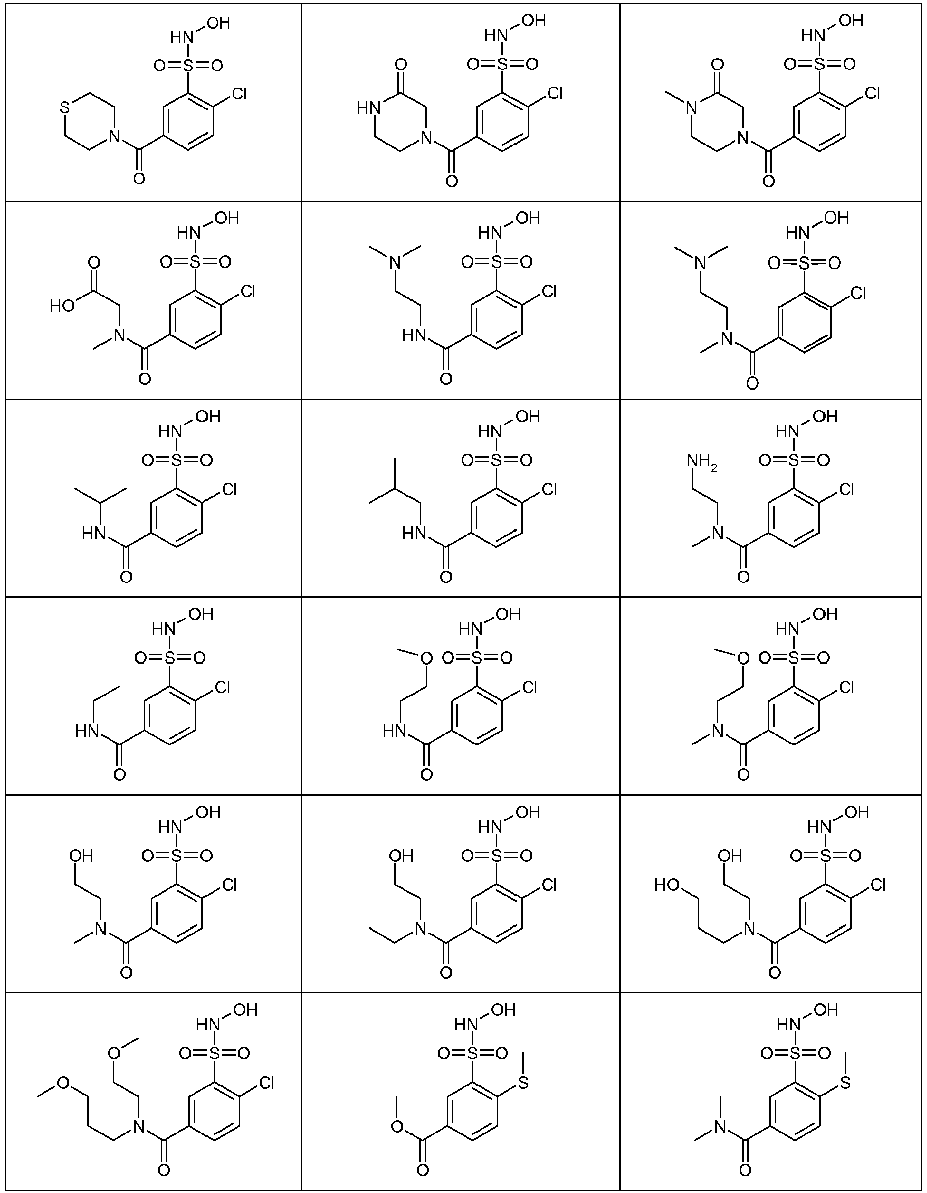

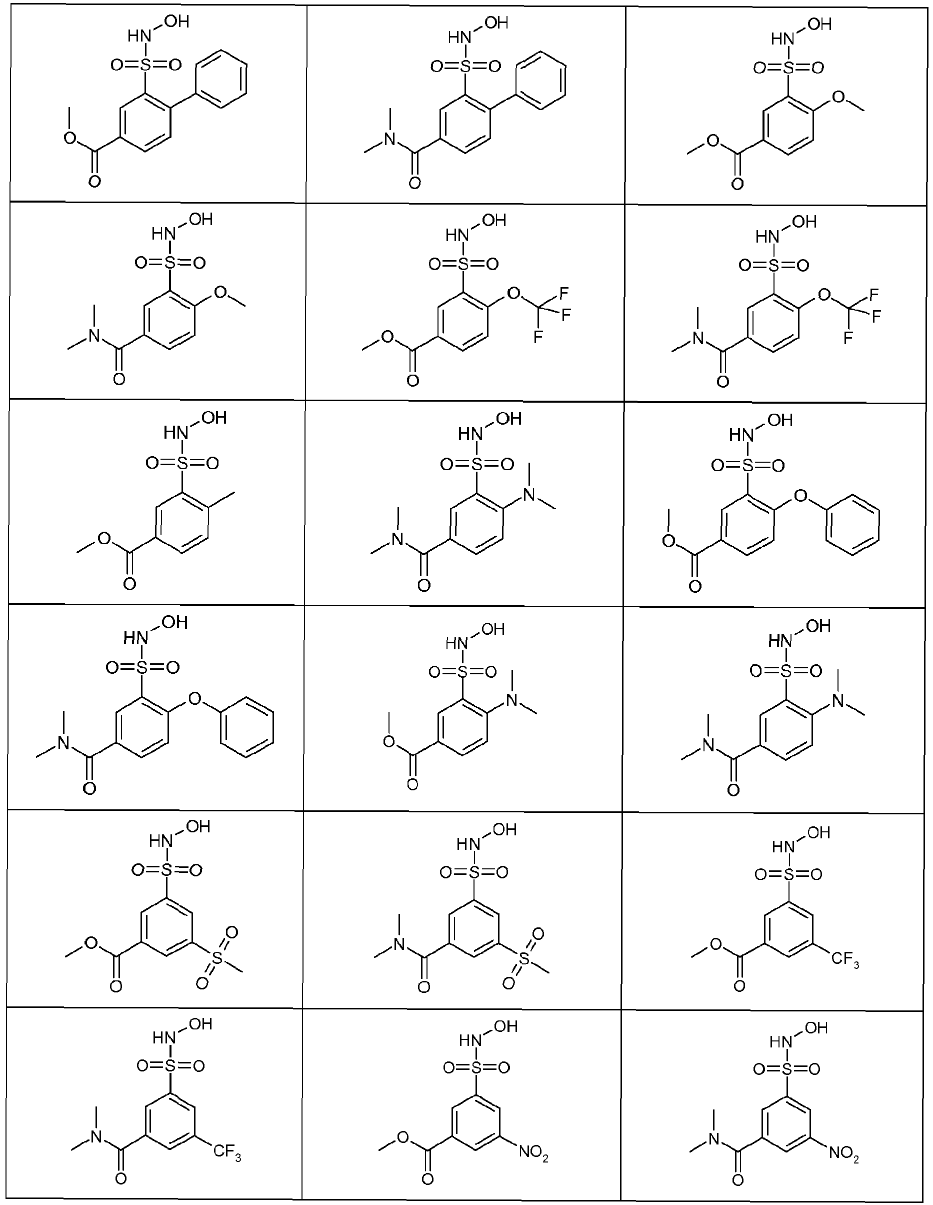

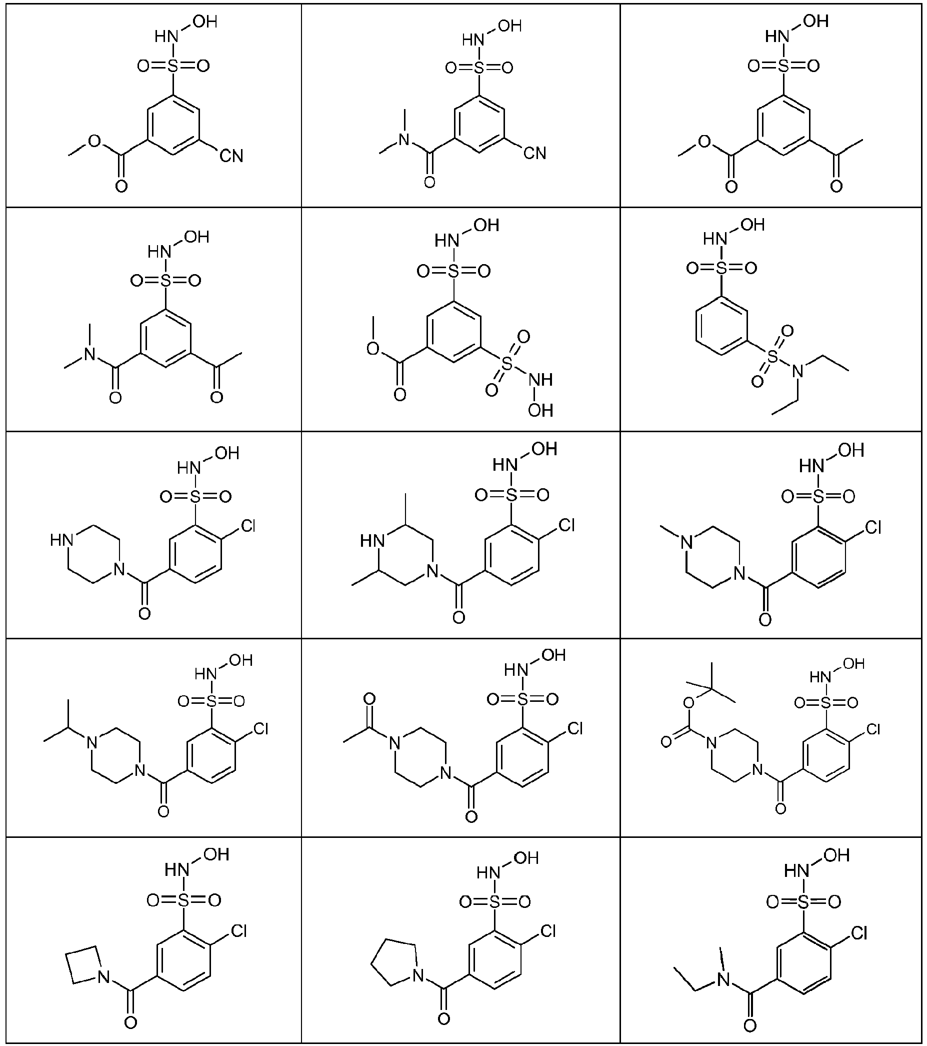

In some embodiments, the HNO donor is a compound of the formula (IIa): 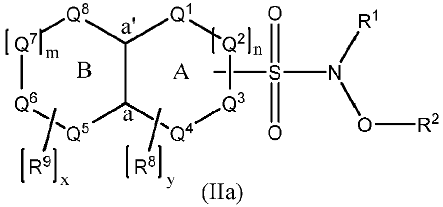 or a pharmaceutically acceptable salt thereof, wherein:
R¹ is H;
R² is H, aralkyl or heterocyclyl;
m and n are independently an integer from 0 to 1;
x is an integer from 0 to 4 and y is an integer from 0 to 3, provided that at least one of x and y is greater than 0;
A is a cycloalkyl, heterocycloalkyl, aromatic or heteroaromatic ring containing ring moieties Q¹, Q², Q³ and Q⁴, which are taken together with the carbons at positions a and a' to form ring A;
B is a cycloalkyl, heterocycloalkyl, aromatic or heteroaromatic ring containing ring moieties Q⁵, Q⁶, Q⁷ and Q⁸, which are taken together with the carbons at positions a and a' to form ring B; Q¹, Q², Q³, Q⁴, Q⁵, Q⁶, Q⁷and Q⁸ are independently selected from C, CH₂, CH, N, NR¹⁰, O and S;
each R⁸ and R⁹ is independently selected from halo, alkylsulfonyl, *N-*hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, NH₂, OH, C(O)OH, C(O)Oalkyl, NHC(O)alkylC(O)OH, C(O)NH₂, NHC(O)alkylC(O)alkyl, NHC(O)alkenylC(O)OH, NHC(O)NH₂, OalkylC(O)Oalkyl, NHC(O)alkyl, C(=N-OH)NH₂, cycloalkoxy, cycloalkylsulfanyl, arylsulfanyl, arylsulfinyl, carbonylamino and sulfonylamino, provided that at least one R⁸ is carbonylamino or sulfonylamino; and
R¹⁰ is H, alkyl, acyl, or sulfonyl.

In some embodiments, the HNO donor is a compound of (IIa), wherein:
each R⁸ and R⁹ is independently selected from Cl, F, I, Br, SO₂CH₃, SO₂NHOH, CF₃, CH₃, NO₂, phenyl, CN, OCH₃, OCF₃, *t*-Bu, O-*i*Pr, 4-nitrophenyloxy (OPh4-NO₂), propane-2-thiyl (SCH(CH₃)₂), propane-2-sulfinyl (S(O)CH(CH₃)₂), morpholino, *N*-methyl-piperazino, dimethylamino, piperidino, cyclohexyloxy, cyclopentylsulfanyl, phenylsulfanyl, phenylsulfinyl, carbonylamino and sulfonylamino, provided that at least one R⁸ is carbonylamino or sulfonylamino; and
R¹⁰ is H, alkyl, acyl or sulfonyl, provided that when rings A and B form naphthalene, x is an integer from 1 to 3 or y is an integer from 2 to 4.

Included in any of the embodiments of formula (IIa) described above are the additional embodiments described below.

In some embodiments, R¹ is H and R² is H, benzyl or tetrahydropyran-2-yl. In some embodiments, A and B form a benzofuran, benzothiophene, benzoimidazole, *N-*alkylbenzoimidazole (such as *N*-methylbenzoimidazole), *N*-acylbenzoimidazole (such as N-C(O)CH₃benzoimidazole), benzothiazole or benzooxazole. In some embodiments, A and B are other than napthyl or quinoline. In some embodiments, A and B are napthyl or quinoline. In some embodiments, A and B form benzofuran. In some embodiments, A and B form a benzofuran. In some embodiments, A and B form benzothiophene. In some embodiments, A and B form benzothiophene, y is 0 and x is 1. In some embodiments, A and B form naphthyl, x is 0 and y is 1. In some embodiments, ring A is phenyl and ring B is heteroaryl, such as when rings A and B form quinoline and ring B is the nitrogen containing ring. The invention also embraces compounds according to any of the embodiments of formula (IIa) wherein y is 0, x is 1 and R⁹ is halo, alkyl or perhaloalkyl. The invention also embraces compounds according to any of the embodiments of formula (IIa) wherein x is 2 and y is 0.

In some embodiments, at least one of R⁸ and R⁹ is -CONH-alkyl. In some embodiments, at least one of R⁸ and R⁹ is -CONR-alkyl wherein R is alkyl. In some embodiments, at least one of R⁸ and R⁹ is -CONR₂ wherein each R is independently alkyl. In some embodiments, y is 0, x is 1 and R⁹ is -CONR₂ wherein each R is independently alkyl. In some embodiments, y is 0, x is 1 and R⁹ is -CONR₂ wherein each R is independently lower alkyl, wherein each lower alkyl can be the same (e.g., -CON(Me)₂) or different. In some embodiments, at least one of R⁸ and R⁹ is -CONR₂ wherein each R taken together with the nitrogen to which it is attached to form a heterocylic or substituted heterocyclic ring. In some embodiments, at least one of R⁸ and R⁹ is -CONR₂ wherein each R is independently alkyl. In some embodiments, at least one of R⁸ and R⁹ is - NR^{a}SO₂NR-alkyl wherein R^{a} and R are independently hydrogen or alkyl. In some embodiments, at least one of R⁸ and R⁹ is -SO₂NH₂. In some embodiments, at least one of R⁸ and R⁹ is -SO₂NH₂. In some embodiments, at least one of R⁸ and R⁹ is -SO₂NR-alkyl, wherein R is hydrogen or alkyl. In some embodiments, at least one of R⁸ and R⁹ is -SO₂NR₂, wherein the two R groups are taken together and with the nitrogen atom to which they are attached to form heterocyclic or substituted heterocyclic. In some embodiments, y is 0, x is 1 and R⁹ is -SO₂NR₂, wherein the two R groups are taken together and with the nitrogen atom to which they are attached to form a heterocyclic ring. In some embodiments, y is 0, x is 1 and R⁹ is -SO₂NR₂, wherein the two R groups are taken together and with the nitrogen atom to which they are attached to form a morpholino group.

Representative compounds of the formula (IIa) include, but are not limited to, the compounds listed in Table 7.

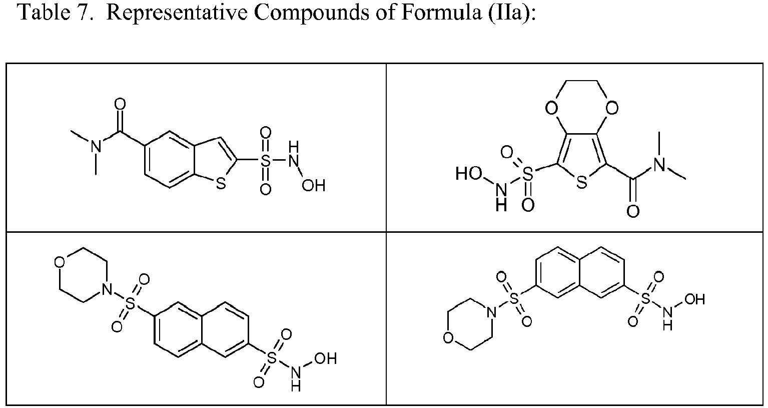

In some embodiments, the HNO donor is a compound of formula (IIIa) 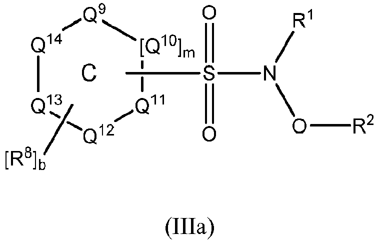 or a pharmaceutically acceptable salt thereof, wherein:
R¹ is H;
R² is H, aralkyl or heterocyclyl;
n is an integer from 0 to 1;
b is an integer from1 to 4;
C is a heteroaromatic ring containing ring moieties Q⁹, Q¹⁰, Q¹¹, Q¹², Q¹³ and Q¹⁴ that are independently selected from C, CH₂, CH, N, NR¹⁰, O and S, provided that at least one of Q⁹, Q¹⁰, Q¹¹, Q¹², Q¹³ and Q¹⁴ is N, NR¹⁰, O or S;
each R⁸ is independently selected from halo, alkylsulfonyl, *N*-hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, NH₂, OH, C(O)OH, C(O)Oalkyl, NHC(O)alkylC(O)OH, C(O)NH₂, NHC(O)alkylC(O)alkyl, NHC(O)alkenylC(O)OH, NHC(O)NH₂, OalkylC(O)Oalkyl, NHC(O)alkyl, C(=N-OH)NH₂, cycloalkoxy, cycloalkylsulfanyl, arylsulfanyl, arylsulfinyl, carbonylamino and sulfonylamino, provided that at least one R⁸ is carbonylamino or sulfonylamino; and
R¹⁰ is H, alkyl, acyl or sulfonyl.

In some embodiments, the HNO donor is a compound of formula (IIIa) and each R⁸ is independently selected from Cl, F, I, Br, SO₂CH₃, SO₂NHOH, CF₃, CH₃, NO₂, phenyl, CN, OCH₃, OCF₃, *t*-Bu, O-*i*Pr, 4-nitrophenyloxy (OPh4-NO₂), propane-2-thiyl (SCH(CH₃)₂), propane-2-sulfinyl (S(O)CH(CH₃)₂), morpholino, *N*-methyl-piperazino, dimethylamino, piperidino, cyclohexyloxy, cyclopentylsulfanyl, phenylsulfanyl, phenylsulfinyl, carbonylamino and sulfonylamino, provided that at least one R⁸ is carbonylamino or sulfonylamino.

In some embodiments, the HNO donor is a compound of the formula (IIIa) and each R⁸ is independently selected from F, Br, Cl, CF₃, phenyl, methyl, SO₂NHOH, morpholino, piperidino, 4-methyl-piperazino, carbonylamino and sulfonylamino, provided that at least one R⁸ is carbonylamino or sulfonylamino.

Included in any of the embodiments of formula (IIIa) described above are the following additional embodiments.

In some embodiments, R¹ is H and R² is H, benzyl or tetrahydropyran-2-yl. In some embodiments, n is 0 and C is a thiophene, isoxazole, pyrazole, pyrrole, imidazole, furan, thiazole, triazole, *N*-methylimidazole or thiadiazole. In some embodiments, C is other than thienyl. In some embodiments, n is 0 and C is a thiophene, isoxazole, pyrazole, pyrrole, imidazole, furan, thiazole, triazole, *N*-methylimidazole or thiadiazole. In some embodiments, n is 1 and C is pyrimidine, pyrazine or pyridine. In some embodiments, n is 1, C is pyrimidine, pyrazine or pyridine, and b is 1. In some embodiments, n is 1, C is pyrimidine, pyrazine or pyridine, b is 1, and at least one R⁸ is chloro, morpholino, piperidino or *N-*methylpiperizino. In some embodiments, C is thiophene and b is 1. In some embodiments, C is thiophene, b is 1 and at least one R⁸ is halo. In some embodiments, C is thiophene.

In some embodiments, at least one R⁸ is -CONH₂. In some embodiments, C is thiophene substituted with -CONH₂, and optionally substituted with an additional R⁸, such as amino. In some embodiments, at least one R⁸ is -CONH-alkyl. In some embodiments, R⁸ is -CONH-lower alkyl (e.g., isopropyl). In some embodiments, at least one R⁸ is -CONH-substituted alkyl. In some embodiments, aC is thiophene, R¹ and R² are both H, and at least one R⁸ is -CONH-substituted alkyl. In some embodiments, at least one R⁸ is -CONR-alkyl wherein R is alkyl. In some embodiments, at least one R⁸ is -CONR₂ wherein each R is independently alkyl, such as -CON(Me)₂. In some embodiments, C is thiophene, b is 2, one of R⁸ is -CONR₂ wherein each R is independently alkyl (such as -CON(Me)₂) and the other R⁸ is -S(O)₂alkyl, aryl, heteroaryl, or -S-alkyl. In some embodiments, at least one R⁸ is - CONR₂ wherein each R is independently a substituted alkyl, such as -CH₂CH₂OCH₃. In some embodiments, at least one R⁸ is -CONR₂ wherein each R taken together with the nitrogen to which it is attached to form a heterocylic or substituted heterocyclic ring. In some embodiments, C is thiophene, R¹ and R² are both H and at least one R⁸ is -CONR₂ wherein each R is taken together with the nitrogen to which it is attached to form a heterocylic or substituted heterocyclic ring, such as morpholino. In some embodiments, C is thiophene, R¹ and R² are both H, b is 1 or 2, at least one R⁸ is -CONR₂ wherein each R is taken together with the nitrogen to which it is attached to form a heterocylic ring selected from piperidinyl and morpholinyl, and when b is 2, the R⁸ that is other than -CONR₂ is selected from halo, nitro and -OR¹¹, such as -Oalkyl (e.g., methoxy). In some embodiments, C is thiophene, R¹ and R² are both H, b is 1 or 2, at least one R⁸ is -CONR₂ wherein each R is taken together with the nitrogen to which it is attached to form a heterocyclic ring substituted with 1 or 2 moieties selected from lower alkyl, carboxylester, acyl, halo, amino, hydroxyl, substituted lower alkyl, oxo and alkoxy. In some embodiments, R⁸ is -CONR₂ wherein each R is taken together with the nitrogen to which it is attached to form a substituted heterocyclic ring selected from 1-methyl-piperaz-4-yl, 4-fluoropiperidyl and 4-hydroxypiperidyl. In some embodiments, when C is thiophene substituted with R⁸ and R is -CONR₂, C may also be substituted with a moiety selected from halo, amino, hydroxyl, alkoxy, nitro and cyano. In some embodiments, at least one R⁸ is -CONR₂ wherein each R is independently alkyl.

In some embodiments, at least one R⁸ is - NR^{a}SO₂NR-alkyl wherein R^{a} and R are independently hydrogen or alkyl. In some embodiments, at least one R⁸ is -SO₂NH₂. In some embodiments, at least one R⁸ is -SO₂NH₂. In some embodiments, at least one R⁸ is -SO₂NR-alkyl, wherein R is hydrogen or alkyl. In some embodiments, at least one R⁸ is -SO₂NR₂, wherein the two R groups are taken together and with the nitrogen atom to which they are attached to form a heterocyclic or substituted heterocyclic ring.

Representative compounds of formula (IIIa) include, but are not limited to, the compounds listed in Table 8.

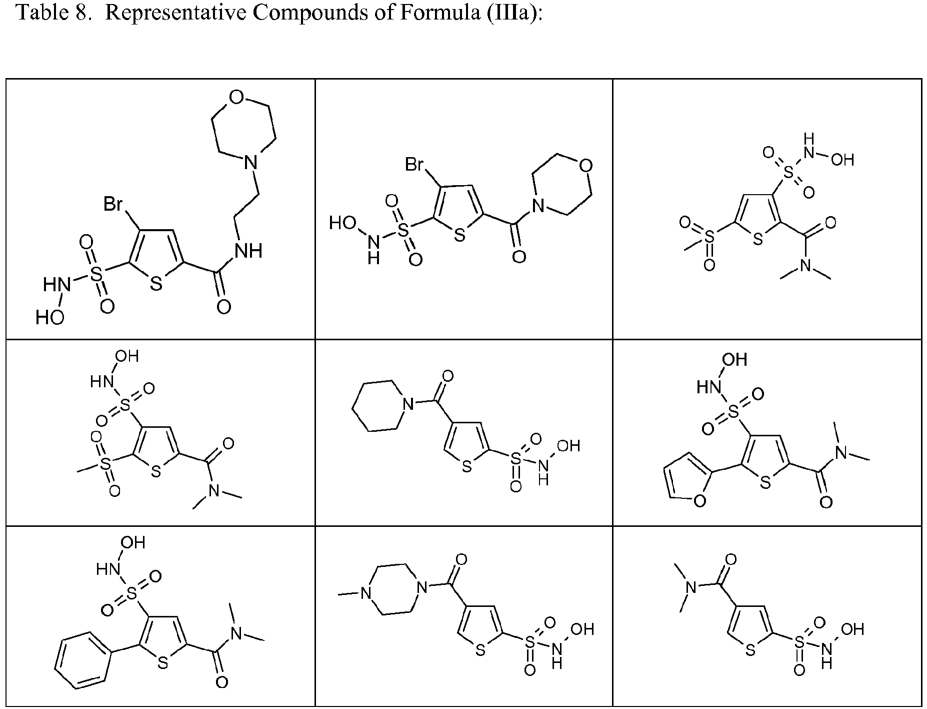

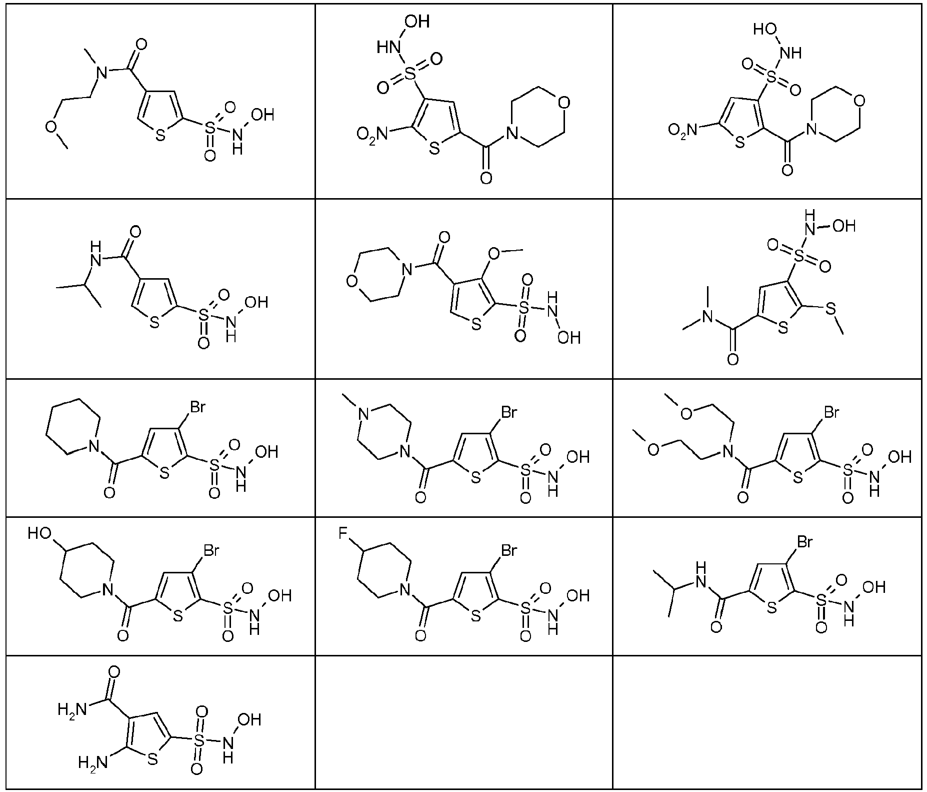

Pharmaceutical compositions that are useful in the methods described herein may comprise an effective amount of one or more nitroxyl donors or pharmaceutically acceptable salts thereof, together with a pharmaceutically acceptable excipient.

The pharmaceutical compositions may be formulated for administration in solid or liquid form, including those adapted for the following: (1) oral administration, for example, drenches (for example, aqueous or non-aqueous solutions or suspensions), tablets (for example, those targeted for buccal, sublingual and systemic absorption), caplets, boluses, powders, granules, pastes for application to the tongue, hard gelatin capsules, soft gelatin capsules, mouth sprays, troches, lozenges, pellets, syrups, suspensions, elixirs, liquids, emulsions and microemulsions; (2) parenteral administration, for example, by subcutaneous, intramuscular, intravenous or epidural injection as, for example, a sterile solution or suspension; (3) topical application, for example, as a cream, ointment, patch, pad or spray applied to the skin; (4) intravaginally or intrarectally, for example, as a pessary, cream or foam; (5) sublingually; (6) ocularly; (7) transdermally; or (8) nasally. The pharmaceutical compositions may be formulated for immediate, sustained or controlled release. In some embodiments, the pharmaceutical compositions are formulated for immediate release. In some embodiments, the pharmaceutical compositions are formulated for sustained release. In some embodiments, the pharmaceutical compositions are formulated for controlled release.In some embodiments, the pharmaceutical compositions are formulated for oral administration. In some embodiments, the pharmaceutical compositions are formulated for intravenous administration. In some embodiments, the pharmaceutical compositions are formulated for administration by inhalation.

Administration of the HNO donor compound, pharmaceutically acceptable salt thereof, or pharmaceutical composition comprising the HNO donor compound or pharmaceutically acceptable salt thereof may be via any accepted mode known to one skilled in the art, for example, orally, parenterally, by inhalation spray, topically, rectally, nasally, buccally, vaginally, intraoccularly, intrapulmonarily, or via an implanted reservoir. The term "parenterally" includes without limitation subcutaneously, intravenously, intramuscularly, intraperitoneally, intrathecally, intraventricularly, intrasternally, intracranially, by intraosseous injection and by infusion techniques. In some embodiments, the compound or pharmaceutical composition is administered intravenously. In some embodiments, the compound or pharmaceutical composition is administered orally.

Any administration regimen well known to those skilled in the art for regulating the timing and sequence of drug delivery can be used and repeated as necessary to effect treatment in the methods disclosed herein. For example, the HNO donor compound or composition may be administered 1, 2, 3 or 4 times daily, by a single dose, multiple discrete doses or continuous infusion.

The administration regimen may include pretreatment and/or co-administration with at least one additional therapeutic agent. In such case, the compound or composition and at least one additional therapeutic agent may be administered simultaneously, separately, or sequentially.

Examples of administration regimens include without limitation:
administration of each compound, composition and therapeutic agent in a sequential manner; and
co-administration of each compound, composition, and therapeutic agent in a substantially simultaneous manner (e.g., as in a single unit dosage form) or in multiple, separate unit dosage forms for each compound, composition, and/or therapeutic agent.

It will be appreciated by those skilled in the art that the "effective amount" or "dose level" will depend on various factors such as the particular administration mode, administration regimen, compound, and composition selected, and the particular disease and patient being treated. For example, the appropriate dose level may vary depending upon the activity, rate of excretion and possible toxicity of the specific compound or composition employed; the age, body weight, general health, gender and diet of the patient being treated; the frequency of administration; the other therapeutic agent(s) being co-administered; and the type and severity of the disease.

The compounds and pharmaceutical compositions disclosed herein may be administered at any suitable dose level, including dose levels on the order of about 0.001 to about 10,000 mg/kg/d. In some embodiments, the dose level is about 0.01 to about 1,000 mg/kg/d. In some embodiments, the dose level is about 0.01 to about 100 mg/kg/d. In some embodiments, the dose level is about 0.01 to about 10 mg/kg/d. In some embodiments, the dose level is about 0.1 to about 1 mg/kg/d. In some embodiments, the dose level is less than about 1 g/kg/d.

Other than in the working examples, or where otherwise indicated, all numbers expressing quantities of ingredients, reaction conditions, and so forth used in the specification and claims are to be understood as being modified by the term "about". Accordingly, unless indicated to the contrary, such numbers are approximations that may vary depending upon the-desired properties sought to be obtained by the present invention. At the very least, and not as an attempt to limit the application of the doctrine of equivalents to the scope of the claims, each numerical parameter should be construed in light of the number of significant digits and ordinary rounding techniques.

While the numerical ranges and parameters setting forth the broad scope of the invention are approximations, the numerical values set forth in the working examples are reported as precisely as possible. Any numerical value, however, inherently contains certain errors necessarily resulting from the standard deviation found in their respective testing measurements.

### EXAMPLES

The following examples are presented for illustrative purposes and should not serve to limit the scope of the invention.

### Example 1: In Vivo Animal Studies (Acute Treatment, Intravenous Infusion)

This example demonstrates the efficacy of HNO donors to lower pulmonary artery pressure in rats with monocrotaline-induced PH.

Rats (200-250 g) are anesthetized via an intra-muscular (i.m.) injection of ketamine/xylazine (80/10 mg/kg). A half dose (40 mg/kg ketamine/5 mg/kg xylazine) is given as supplemental anesthesia as needed. Animals are placed on a heating pad set to maintain body temperature at approximately 37°C. Body temperature is monitored throughout the experiment. Once consciousness is lost, a pressure transducer is inserted into a femoral artery to measure arterial blood pressure. A fluid filled catheter is inserted through the right jugular vein into the pulmonary artery to measure pulmonary artery pressure via a pressure transducer. A cannula is placed into the left jugular vein for dosing.

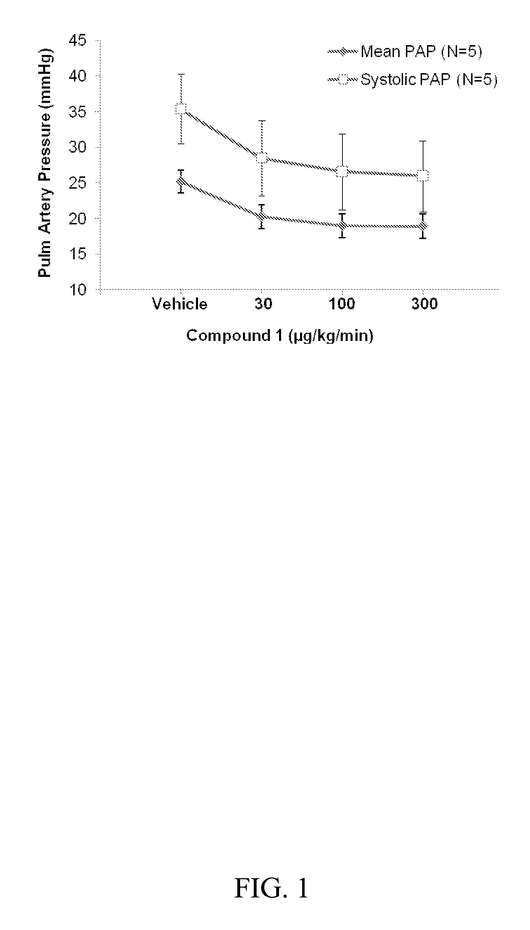

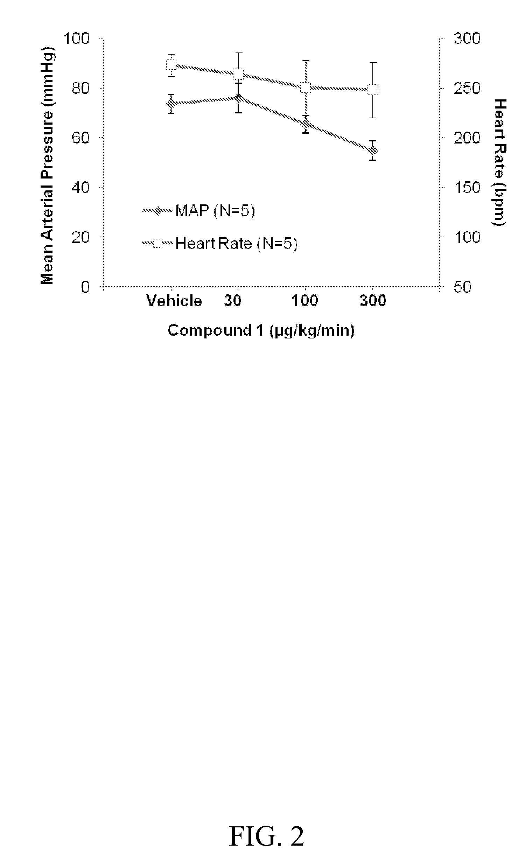

Monocrotaline is administered via a single subcutaneous injection (60 mg/kg) approximately 3 weeks prior to the terminal procedure. A baseline pulmonary artery pressure of >30 mmHg is required to initiate study of the compounds described herein. An HNO donor is administered intravenously in a dose-escalation manner in 20 minute intervals from doses of 10 to 300 µg/kg/min. Hemodynamic indices, including MAP (mean arterial pressure), SAP (systolic arterial pressure), DAP (diastolic arterial pressure), HP (heart rate), MPAP (mean pulmonary arterial pressure), SPAP (systolic pulmonary arterial pressure), DPAP (diastolic pulmonary arterial pressure), are measured. The results are illustrated in FIG. 1 and FIG. 2.

For the terminal procedure, after surgical instrumentation and an approximate 10 minute pre-dose equilibration period, HNO donor solutions are infused via jugular vein catheter. At the end of the experiment, rats are euthanized under anesthesia via pentobarbital overdose.

**Example 2:** *In Vivo* Animal Studies (Acute Treatment, Intravenous Infusion or Inhaled Administration)

This example demonstrates the efficacy of HNO donors to lower pulmonary artery pressure in dogs with hypoxia-induced PH.

Healthy dogs (10-15 kg) are anesthetized with pentobarbital (20-40 mg/kg. intravenously) and anesthesia is maintained by continuous infusion of pentobarbital at rate of 5-10 mg/kg/h. Dogs are intubated via a tracheotomy, and artificially respired (while monitoring inspired oxygen and expired CO₂). The left femoral vein and artery are cannulated for dose administration and arterial blood pressure recording. The right jugular vein is cannulated with a pulmonary artery pressure catheter (Swan Ganz catheter), to measure both pulmonary arterial pressure (PAP) and pulmonary wedge pressure (PWP). This catheter is also used for measurement of cardiac output via thermodilution techniques following rapid injection of cold 5 mL saline. Electrocardiograms are monitored throughout the experiment.

During the baseline and control measurements inspired oxygen is maintained at 40%. Hypoxia is induced by adding nitrogen to the respiratory gas at a rate sufficient to reduce respired oxygen to 10% (FiO2=10%). Each hypoxic condition is maintained for 15-30 minutes and then normoxic (Fi02=40%) control condition is returned. Each dose of HNO donor is intravenously administered during the 30 minute hypoxic condition; no drug is infused during the subsequent normoxia until the next dose is given. HNO donors are given intravenously in the range of 1 to 100 µg/kg/min and various hemodynamic indices are recorded. Alternatively, in this experiment HNO donors are administered using an inhalation nebulizer at dose levels of 0.1-1 g/kg in 5-10 time period during each hypoxia period.

### Example 3: In Vivo Animal Studies (Chronic Treatment, Continuous Intravenous Infusion)

This example demonstrates the efficacy of HNO donors to retard the progression of disease in rats with monocrotaline-induced PH.

Rats (200-250g) are surgically implanted with a pressure transducer equipped telemetry transmitter. The transmitter assembly is secured internally; the fluid-filled catheter is placed into the jugular vein with the tip of the pressure transducer positioned in the right ventricle for collection of right ventricular pressure (RVP) data. Additionally, all animals, with the exception of the sham group, are implanted with femoral vein cannulas for the purposes of dosing.

Monocrotaline (MCT) is administered to vehicle-control animals by subcutaneous injection. One week following the MCT injection, the vehicle-control animals are administered saline or a low or high dose of an HNO donor by continuous intravenous infusion for two weeks. The test and vehicle control article are administered by external pump. Weekly clinical observations are performed on animals.

For cardiovascular evaluations, RVP data is collected with animals allowed free movement in the home cage. The animals are monitored for at least 24 hours prior to MCT administration. RVP is also monitored at 24 hours following the end of the two week infusion, and occurs for at least 24 hours. All animals are necropsied at the end of the study. Weights of lungs and pulmonary artery, heart and each individual chamber are evaluated. The weights of the heart, LV, RV, and ratio to body weight are reported. The small pulmonary arteries from each animal are evaluated for medial thickness, neointima, and smooth muscle hypertrophy.

### Example 4: In Vivo Animal Studies (Chronic Treatment, Oral Administration)

This example demonstrates the efficacy of HNO donors to retard the progression of disease in rats with monocrotaline-induced PH.

The general methods for this experiment is similar to that of Example 3 above. One difference is that the route of administration is oral, with a dosing regimen of once to four times daily at dose levels of 0.1-1 g/kg.

### Example 5: In Vivo Animal Studies (Chronic Treatment, Continuous Intravenous Infusion)

This example demonstrates the efficacy of HNO donors to reverse the progression of disease in rats with monocrotaline-induced PH.

In this study, rats (200-250g) rats are surgically implanted with a pressure transducer equipped telemetry transmitter. The transmitter assembly is secured internally; the fluid-filled catheter is placed into the jugular vein with the tip of the pressure transducer positioned in the right ventricle for collection of right ventricular pressure (RVP) data. Additionally, all animals, with exception of sham group, are implanted with femoral vein cannulas for the purposes of dosing.

The vehicle and control article, Monocrotaline (MCT), are administered by subcutaneous injection. Three weeks following the MCT injection, animals are administered saline or a low or high dose of an HNO donor by continuous intravenous infusion for three weeks. The HNO donor and vehicle control article are administered by external pump. Weekly clinical observations are performed on the animals.

For cardiovascular evaluations, RVP data is collected with animals allowed free movement in the home cage. The animals are monitored for at least 24 hours prior to MCT administration. RVP is also monitored for at least 24 hours following the end of the two week infusion. All animals are necropsied at the end of the study. Weights of lungs and pulmonary artery, heart and each individual chamber are evaluated. The weights of the heart, LV, RV, and ratio to body weight are reported. The small pulmonary arteries from each animal are evaluated for medial thickness, neointima, and smooth muscle hypertrophy.

### Example 6: In Vivo Animal Studies (Chronic Treatment, Oral Administration)

This example demonstrates the efficacy of HNO donors to reverse the progression of disease in rats with monocrotaline-induced PH.

The general methodology is similar to that of Example 5, with the exception that the route of administration is oral, with a dosing regimen of one to four times daily at dose levels of 0.1-1 g/kg.

### Example 7: In Vivo Animal Studies (Chronic Treatment, Inhaled Administration)

This example demonstrates the efficacy of HNO donors to retard progression of disease in rats with monocrotaline-induced PH.

The general methodology is similar to that of Example 3 above, with the exception that the route of administration is via inhalation, with a dosing regimen of one to four times daily at dose levels of 0.1-1 g/kg.

### Example 8: In Vivo Animal Studies (Chronic Treatment, Inhaled Administration)

This example demonstrates the efficacy of HNO donors to reverse the progression of disease in rats with monocrotaline-induced PH.

The general methodology is similar to that of Example 3, with the exception that the route of administration is via inhalation, with a dosing regimen of one to four times daily at dose levels of 0.1-1 g/kg.

### Example 9: In Vivo Animal Studies (Acute Treatment, Intravenous Infusion and Inhaled Administration)

This example demonstrates the efficacy of HNO donors to lower pulmonary artery pressure in dogs with thromboxane-induced PH.

Experimental PH is induced by continuous infusion of a thromboxane A2 receptor agonist analog (for example U46619, Tocris Bioscience). The thromboxane A2 receptor agonist analog infusion rate (0.1-1 mg/kg/min) is adjusted to maintain a systolic pulmonary artery pressure (PAP) at 40 mmHg in anesthetized and mechanically ventilated dogs. The left femoral vein and artery are cannulated for dose administration and arterial blood pressure recording. The right jugular vein is cannulated with a pulmonary artery pressure catheter (Swan Ganz catheter), to measure both pulmonary arterial pressure (PAP) and pulmonary wedge pressure (PWP). This catheter is also used for measurement of cardiac output via thermodilution techniques following rapid injection of cold 5 mL saline. Electrocardiograms are monitored throughout the experiment.

Once a stable steady-state in hemodynamic is achieved, various doses of HNO donors are given intravenously at dose rates in the range of 1 to 100 µg/kg/min and various hemodynamic indices are recorded. Alternatively, in this experiment HNO donors are administered using an inhalation nebulizer at dose levels of 0.1-1 g/kg in 5-10 minute time period.

### Example 10: In Vivo Human Studies (Acute Treatment, Intravenous Infusion and Inhaled Administration)

This example demonstrates the efficacy of HNO donors to lower pulmonary artery pressure in human subjects with various causes of pulmonary hypertension.

Patients (either gender) with various causes of pulmonary hypertension are selected for this study. Baseline hemodynamic characteristics of the patients are assessed by collected various hemodynamic indices utilizing right heart catheterization (e.g. right atrial pressure, mean pulmonary artery pressure, cardiac index), and blood gas profiling. Cardiac rhythm is monitored using continuous electrocardiography, and arterial pressure is monitored using a pressure cuff. Patients are tested for reversibility of pulmonary hypertension using nitric oxide (NO) by inhalation. Hemodynamic indices are then reassessed. Once all indices have returned to baseline upon cessation of NO delivery, and a baseline is established, various doses of HNO donors are given intravenously at dose rates in the range of 1 to 100 µg/kg/min (either continuous dose or in a dose-escalation fashion) and various hemodynamic indices are recorded. Alternatively, in this experiment HNO donors are administered using an inhalation nebulizer at dose levels of 0.1-1 g/kg in 5-10 minute time period. Hemodynamic indices are assessed at various time points during the infusion period. A few patients receive placebo instead of HNO donor in a double-blind randomized fashion. From the data collected during various periods of the trial, the pulmonary and systemic vascular resistances are calculated.

It will be apparent to those skilled in the art that specific embodiments of the invention may be directed to one, some or all of the above- and below-indicated embodiments in any combination.

## Claims

1. A nitroxyl donor for use in a method of treating pulmonary hypertension, which method comprises administering to a mammal in need thereof an effective amount of the nitroxyl donor, wherein the nitroxyl donor is a compound of formula I 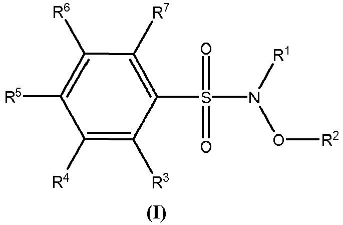 or a pharmaceutically acceptable salt thereof, wherein:
R¹ is H;
R² is H;
R³, R⁴, R⁵, R⁶ and R⁷ are independently selected from H, halo, alkylsulfonyl, *N-*hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, cycloalkoxy, cycloalkylsulfanyl, arylsulfanyl and arylsulfinyl.

2. The nitroxyl donor of claim 1 for use in a method as defined in claim 1, wherein the effective amount is less than about 1 g/kg/d and/or wherein the mammal is human.

3. The nitroxyl donor of claim 1 for use in a method as defined in claim 1 or 2, wherein the compound is administered intravenously, orally, or by inhalation.

4. The nitroxyl donor of claim 1 for use in a method as defined in any one of claims 1 to 3, wherein the pulmonary hypertension is pulmonary arterial hypertension, chronic thromboembolic pulmonary hypertension, or pulmonary hypertension owing to left heart disease, wherein the left heart disease is optionally left heart failure, and wherein the left heart failure is optionally (i) systolic heart failure, (ii) diastolic heart failure, or (iii) chronic or acutely decompensated.

5. The nitroxyl donor of claim 1 for use in a method as defined in any one of claims 1 to 4, wherein:
(1) one of R³ and R⁷ is other than H; at least one of R³, R⁴, R⁵, R⁶ and R⁷ is other than halo; when one of R³ or R⁷ is halo and the R³ or R⁷ that is not halo is H and one of R⁴ or R⁶ is halo and the R⁴ or R⁶ that is not halo is H, R⁵ is other than halo or hydrogen; when R⁴, R⁵ and R⁶ are H and one of R³ and R⁷ is H, the R³ or R⁷ that is not H is other than nitro or alkyl; and when R⁴ and R⁶ are H and R³ and R⁷ are alkyl, R⁵ is other than alkyl; or
(2) R³ is halo, alkylsulfonyl, perhaloalkyl, alkyl having 1 to 4 carbon atoms, nitro or cyano; or
(3) R³ is halo, alkylsulfonyl, perhaloalkyl, alkyl having 1 to 4 carbon atoms, nitro or cyano; and at least three of R⁴, R⁵, R⁶ and R⁷ are H; or
(4) R³ is halo, alkylsulfonyl, perhaloalkyl, alkyl having 1 to 4 carbon atoms, nitro or cyano; and R⁴, R⁵, R⁶ and R⁷ are H; or
(5) R³ is halo, methylsulfonyl, perfluoromethyl, perfluoromethoxy, isopropyl, nitro or cyano; or
(6) R³ is halo, methylsulfonyl, perfluoromethyl, perfluoromethoxy, isopropyl, nitro or cyano; and at least three of R⁴, R⁵, R⁶ and R⁷ are H; or
(7) R³ is halo, methylsulfonyl, perfluoromethyl, perfluoromethoxy, isopropyl, nitro or cyano; and R⁴, R⁵, R⁶ and R⁷ are H.

6. The nitroxyl donor of claim 1 for use in a method as defined in any one of claims 1 to 4, wherein the nitroxyl donor is a compound selected from:
2,6-Dichloro-*N*-hydroxy benzene sulfonamide;
2-Bromo-4-fluoro-*N*-hydroxy benzene sulfonamide;
2,5-Di-trifluoromethyl-*N*-hydroxy benzene sulfonamide;
2-Chloro-4-fluoro-*N*-hydroxy benzene sulfonamide;
2,3-Dichloro-*N*-hydroxy benzene sulfonamide;
2-Chloro-4-bromo-*N*-hydroxy benzene sulfonamide;
2-Nitro-4-trifluoromethyl-*N*-hydroxy benzene sulfonamide;
2-Iodo-*N*-hydroxy benzene sulfonamide;
*N*-Hydroxy-2-methanesulfonyl benzene sulfonamide;
2,4-Di-bromo-*N*-hydroxy benzene sulfonamide;
2-Chloro-4-trifluoromethyl-*N*-hydroxy benzene sulfonamide;
2,4,6-Tri-isopropyl-*N*-hydroxy benzene sulfonamide;
2,4-Di-fluoro-*N*-hydroxy benzene sulfonamide;
2-Fluoro-*N*-hydroxy benzene sulfonamide;
2-Bromo-*N*-hydroxy benzene sulfonamide;
2-(Trifluoromethyl)-*N*-hydroxy benzene sulfonamide;
*N*-Hydroxy-2-phenyl benzene sulfonamide; and
pharmaceutically acceptable salts thereof.

7. The nitroxyl donor of claim 1 for use in a method as defined in any one of claims 1 to 4, wherein the compound is 2-Iodo-*N*-hydroxy benzene sulfonamide, *N*-Hydroxy-2-methanesulfonyl benzene sulfonamide, 2-Fluoro-*N*-hydroxy benzene sulfonamide, 2-Chloro-*N*-hydroxy benzene sulfonamide, 2-Bromo-*N*-hydroxy benzene sulfonamide, or 2-(Trifluoromethyl)-*N*-hydroxy benzene sulfonamide.

8. The nitroxyl donor of claim 1 for use in a method as defined in any one of claims 1 to 4, wherein the nitroxyl donor is a compound of formula (I): 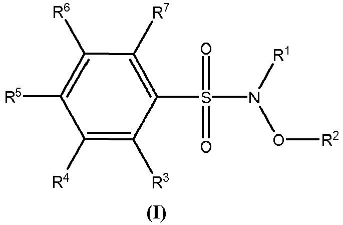 or a pharmaceutically acceptable salt thereof, wherein:
R¹ is H;
R² is H;
R³, R⁴, R⁵, R⁶, and R⁷ are independently selected from H, halo, alkylsulfonyl, N-hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, cycloalkoxy, cycloalkylsulfanyl, arylsulfanyl, and arylsulfinyl, provided that at least one of R³, R⁴, R⁵, R⁶, and R⁷ is other than H; and that at least one of R³, R⁴, R⁵, R⁶, and R⁷ is other than halo; and
wherein R³, R⁵, and R⁷ are H, one of R⁴ and R⁶ is H, and the R⁴ or R⁶ that is not H is other than N-hydroxysulfonamidyl, perhaloalkyl or nitro.

9. The nitroxyl donor of claim 1 for use in a method as defined in any one of claims 1 to 4, wherein the nitroxyl donor is:

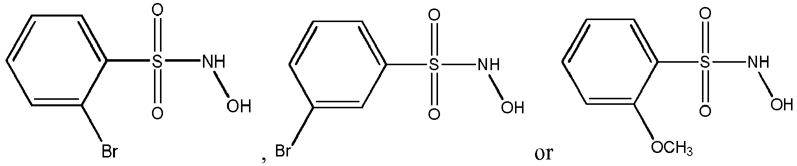

10. A nitroxyl donor for use in a method of treating pulmonary hypertension, which method comprises administering to a mammal in need thereof an effective amount of the nitroxyl donor, wherein the nitroxyl donor is a compound of formula (I): 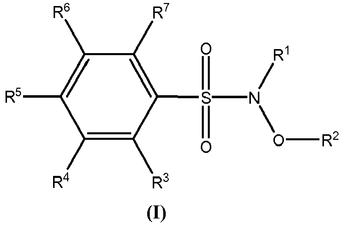 or a pharmaceutically acceptable salt thereof, wherein:
R¹ is H;
R² is H, benzyl, or tetrahydropyran-2-yl; and
R³, R⁴, R⁵, R⁶, and R⁷ are independently selected from H, Cl, F, I, Br, SO₂CH₃, SO₂NHOH, CF₃, NO₂, phenyl, CN, OCH₃, OCF₃, *t*-butyl, O-isopropyl, 4-nitrophenyloxy, propane-2-thiyl, propane-2-sulfinyl, morpholino, *N*-methyl-piperazino, dimethylamino, piperidino, cyclohexyloxy, cyclopentylsulfanyl, phenylsulfanyl, phenylsulfinyl, carboxyl, carboxyl ester, acylamino and sulfonylamino, provided that at least one of R³, R⁴, R⁵, R⁶, and R⁷ is sulfonylamino.

11. A nitroxyl donor for use in a method of treating pulmonary hypertension, which method comprises administering to a mammal in need thereof an effective amount of the nitroxyl donor, wherein the nitroxyl donor is a compound of formula (III) 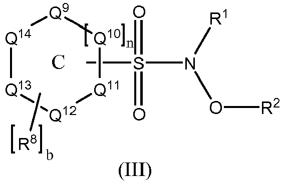 or a pharmaceutically acceptable salt thereof, wherein:
R¹ is H;
R² is H, aralkyl, or heterocyclyl;
Q⁹ Q¹⁰, Q¹¹, Q¹², Q¹³, Q¹⁴ and n are defined such that ring C is thiophene;
b is 1;
R⁸ is halo, alkylsulfonyl, *N*-hydroxylsulfonamidyl, perhaloalkyl, nitro, aryl, cyano, alkoxy, perhaloalkoxy, alkyl, substituted aryloxy, alkylsulfanyl, alkylsulfinyl, heterocycloalkyl, substituted heterocycloalkyl, dialkylamino, NH₂, OH, C(O)OH, C(O)Oalkyl, NHC(O)alkylC(O)OH, C(O)NH₂, NHC(O)alkylC(O)alkyl, NHC(O)alkenylC(O)OH, NHC(O)NH₂, O-alkylC(O)Oalkyl, NHC(O)alkyl, C(=N-OH)NH₂, cycloalkoxy, cycloalkylsulfanyl, arylsulfanyl, or arylsulfinyl; and
halo is F, Cl, Br, or I.

12. A nitroxyl donor for use in a method of treating pulmonary hypertension, which method comprises administering to a mammal in need thereof an effective amount of the nitroxyl donor, wherein the nitroxyl donor is a compound of formula (III): 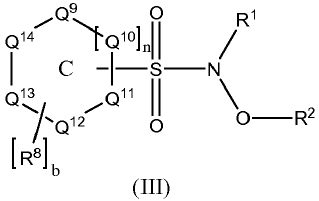 or a pharmaceutically acceptable salt thereof, wherein:
R¹ is H;
R² is H, benzyl, or tetrahydropyran-2-yl;
n is 0;
Q⁹ Q¹⁰, Q¹¹, Q¹², Q¹³ and Q¹⁴ are independently selected from C, CH₂, CH, N, NR¹⁰, O, and S such that C is a thiophene, isoxazole, pyrazole, pyrrole, imidazole, furan, thiazole, triazole, N-methylimidazole, or thiadiazole;
R¹⁰ is H, alkyl, acyl, or sulfonyl;
either (1) b is 1 and R⁸ is halo, nitro, alkyl, or cyano or (2) b is 2 and each R⁸ is independently a halo; and
halo is F, Cl, Br, or I.

13. A nitroxyl donor for use in a method of treating pulmonary hypertension, which method comprises administering to a mammal in need thereof an effective amount of the nitroxyl donor, wherein the nitroxyl donor is a compound of formula (III): 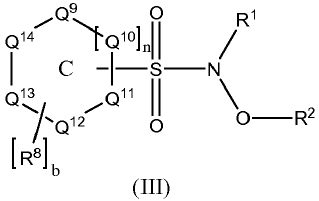 or a pharmaceutically acceptable salt thereof, wherein:
R¹ is H;
R² is H, aralkyl, or heterocyclyl;
n is 0;
Q⁹ Q¹⁰, Q¹¹, Q¹², Q¹³ and Q¹⁴ are independently selected from C, CH₂, CH, N, NR¹⁰, O, and S such that C is a thiophene, isoxazole, pyrazole, pyrrole, imidazole, furan, thiazole, triazole, N-methylimidazole, or thiadiazole;
R¹⁰ is H, alkyl, acyl, or sulfonyl;
b is 1 and R⁸ is halo, nitro, alkyl, or cyano; and
halo is F, Cl, Br, or I.

14. A nitroxyl donor for use in a method of treating pulmonary hypertension, which method comprises administering to a mammal in need thereof an effective amount of the nitroxyl donor, wherein the nitroxyl donor is:

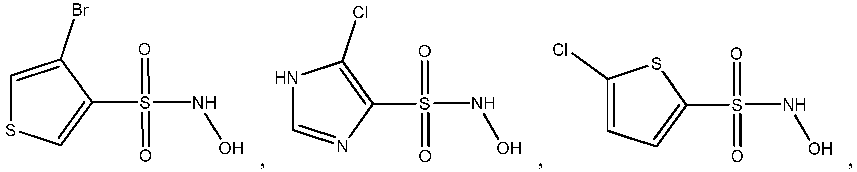

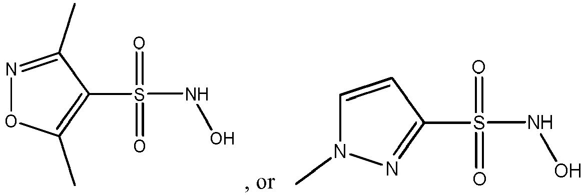

15. A nitroxyl donor for use in a method of treating pulmonary hypertension, which method comprises administering to a mammal in need thereof an effective amount of the nitroxyl donor, wherein the nitroxyl donor is a compound of formula (II): 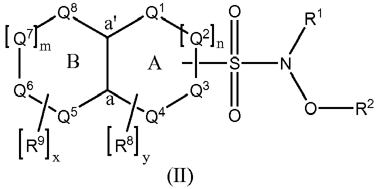 or a pharmaceutically acceptable salt thereof, wherein:
R¹ is H;
R² is H, aralkyl, or heterocyclyl;
m and n are independently an integer from 0 to 1;
A is a cycloalkyl, heterocycloalkyl, aromatic or heteroaromatic ring containing ring moieties Q¹, Q², Q³ and Q⁴, which are taken together with the carbons at positions a and a' to form ring A;
B is a cycloalkyl, heterocycloalkyl, aromatic or heteroaromatic ring containing ring moieties Q⁵, Q⁶, Q⁷ and Q⁸, which are taken together with the carbons at positions a and a' to form ring B;
Q¹, Q², Q³ Q⁴, Q⁵, Q⁶, Q⁷ and Q⁸ are independently selected from C, CH₂, CH, N, NR¹⁰, O and S, provided that when rings A and B form naphthalene, x is an integer from 1 to 3 or y is an integer from 2 to 3;
x is an integer from 0 to 4;
y is an integer from 0 to 3;
each R⁸ and R⁹ is independently selected from Cl, F, I, Br, SO₂CH₃, SO₂NHOH, CF₃, CH₃, NO₂, phenyl, CN, OCH₃, OCF₃, *t*-butyl, O-isopropyl, 4-nitrophenyloxy, propane-2-thiyl, propane-2-sulfinyl, morpholino, *N*-methyl-piperazino, dimethylamino, piperidino, cyclohexyloxy, cyclopentylsulfanyl, phenylsulfanyl, and phenylsulfinyl; and
R¹⁰ is H, alkyl, acyl or sulfonyl.

16. The nitroxyl donor of claim 15 for use in a method as defined in claim 15, wherein the nitroxyl donor is:

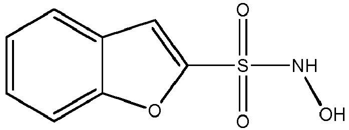

## Patentansprüche

1. Nitroxyldonator zur Verwendung in einem Verfahren zum Behandeln von pulmonaler Hypertonie, welches Verfahren umfasst, an einen Säuger, der dessen bedarf, eine wirksame Menge von dem Nitroxyldonator zu verabreichen, wobei der Nitroxyldonator eine Verbindung der Formel I 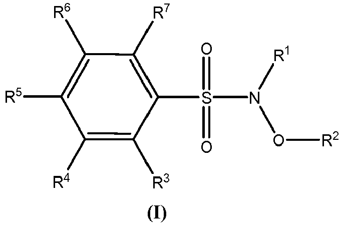 oder ein pharmazeutisch verträgliches Salz davon ist, wobei:
R¹ H ist;
R² H ist;
R³, R⁴, R⁵, R⁶ und R⁷ unabhängig aus H, Halogen, Alkylsulfonyl, *N-*Hydroxylsulfonamidyl, Perhalogenalkyl, Nitro, Aryl, Cyano, Alkoxy, Perhalogenalkoxy, Alkyl, substituiertem Aryloxy, Alkylsulfanyl, Alkylsulfinyl, Heterocycloalkyl, substituiertem Heterocycloalkyl, Dialkylamino, Cycloalkoxy, Cycloalkylsulfanyl, Arylsulfanyl und Arylsulfinyl ausgewählt sind.

2. Nitroxyldonator nach Anspruch 1 zur Verwendung in einem Verfahren, wie in Anspruch 1 definiert, wobei die wirksame Menge weniger als etwa 1 g/kg/d beträgt und/oder wobei der Säuger ein Mensch ist.

3. Nitroxyldonator nach Anspruch 1 zur Verwendung in einem Verfahren, wie in Anspruch 1 oder 2 definiert, wobei die Verbindung intravenös, oral oder durch Inhalation verabreicht wird.

4. Nitroxyldonator nach Anspruch 1 zur Verwendung in einem Verfahren, wie in einem der Ansprüche 1 bis 3 definiert, wobei die pulmonale Hypertonie pulmonalarterielle Hypertonie, chronische thromboembolische pulmonale Hypertonie oder pulmonale Hypertonie infolge Linksherzkrankheit ist, wobei die Linksherzkrankheit gegebenenfalls Linksherzversagen ist und wobei das Linksherzversagen gegebenenfalls (i) systolisches Herzversagen, (ii) diastolisches Herzversagen oder (iii) chronisch oder akut dekompensiert ist.

5. Nitroxyldonator nach Anspruch 1 zur Verwendung in einem Verfahren, wie in einem der Ansprüche 1 bis 4 definiert, wobei:
(1) eines von R³ und R⁷ anders als H ist; mindestens eines von R³, R⁴, R⁵, R⁶ und R⁷ anders als Halogen ist; wenn eines von R³ oder R⁷ Halogen ist und das R³ oder R⁷, das nicht Halogen ist, H ist und eines von R⁴ oder R⁶ Halogen ist und das R⁴ oder R⁶, das nicht Halogen ist, H ist, R⁵ anders als Halogen oder Wasserstoff ist; wenn R⁴, R⁵ und R⁶ H sind und eines von R³ und R⁷ H ist, das R³ oder R⁷, das nicht H ist, anders als Nitro oder Alkyl ist; und wenn R⁴ und R⁶ H sind und R³ und R⁷ Alkyl sind, R⁵ anders als Alkyl ist; oder
(2) R³ Halogen, Alkylsulfonyl, Perhalogenalkyl, Alkyl mit 1 bis 4 Kohlenstoffatomen, Nitro oder Cyano ist; oder
(3) R³ Halogen, Alkylsulfonyl, Perhalogenalkyl, Alkyl mit 1 bis 4 Kohlenstoffatomen, Nitro oder Cyano ist; und mindestens drei von R⁴, R⁵, R⁶ und R⁷ H sind; oder
(4) R³ Halogen, Alkylsulfonyl, Perhalogenalkyl, Alkyl mit 1 bis 4 Kohlenstoffatomen, Nitro oder Cyano ist; und R⁴, R⁵, R⁶ und R⁷ H sind; oder
(5) R³ Halogen, Methylsulfonyl, Perfluormethyl, Perfluormethoxy, Isopropyl, Nitro oder Cyano ist; oder
(6) R³ Halogen, Methylsulfonyl, Perfluormethyl, Perfluormethoxy, Isopropyl, Nitro oder Cyano ist; und mindestens drei von R⁴, R⁵, R⁶ und R⁷ H sind; oder
(7) R³ Halogen, Methylsulfonyl, Perfluormethyl, Perfluormethoxy, Isopropyl, Nitro oder Cyano ist; und R⁴, R⁵, R⁶ und R⁷ H sind.

6. Nitroxyldonator nach Anspruch 1 zur Verwendung in einem Verfahren, wie in einem der Ansprüche 1 bis 4 definiert, wobei der Nitroxyldonator eine Verbindung ist, ausgewählt aus:
2,6-Dichlor-*N*-hydroxybenzolsulfonamid;
2-Brom-4-fluor-*N*-hydroxybenzolsulfonamid;
2,5-Di-trifluormethyl-*N*-hydroxybenzolsulfonamid;
2-Chlor-4-fluor-*N*-hydroxybenzolsulfonamid;
2,3-Dichlor-*N*-hydroxybenzolsulfonamid;
2-Chlor-4-brom-*N*-hydroxybenzolsulfonamid;
2-Nitro-4-trifluormethyl-*N*-hydroxybenzolsulfonamid;
2-Iod-*N*-hydroxybenzolsulfonamid;
*N*-Hydroxy-2-methansulfonyl-benzolsulfonamid;
2,4-Di-brom-*N*-hydroxybenzolsulfonamid;
2-Chlor-4-trifluormethyl-*N*-hydroxybenzolsulfonamid;
2,4,6-Tri-isopropyl-*N*-hydroxybenzolsulfonamid;
2,4-Di-fluor-*N*-hydroxybenzolsulfonamid;
2-Fluor-*N*-hydroxybenzolsulfonamid,
2-Brom-*N*-hydroxybenzolsulfonamid,
2-(Trifluormethyl)-*N*-hydroxybenzolsulfonamid;
*N*-Hydroxy-2-phenylbenzolsulfonamid; und
pharmazeutisch verträgliche Salze davon.

7. Nitroxyldonator nach Anspruch 1 zur Verwendung in einem Verfahren, wie in einem der Ansprüche 1 bis 4 definiert, wobei die Verbindung 2-Iod-*N-*hydroxybenzolsulfonamid, *N*-Hydroxy-2-methansulfonylbenzolsulfonamid, 2-Fluor-*N-*hydroxybenzolsulfonamid, 2-Chlor-*N*-hydroxybenzolsulfonamid, 2-Brom-*N-*hydroxybenzolsulfonamid oder 2-(Trifluormethyl)-*N*-hydroxybenzolsulfonamid ist.

8. Nitroxyldonator nach Anspruch 1 zur Verwendung in einem Verfahren, wie in einem der Ansprüche 1 bis 4 definiert, wobei der Nitroxyldonator eine Verbindung der Formel (I) ist: 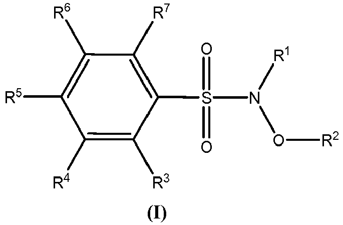 oder ein pharmazeutisch verträgliches Salz davon, wobei:
R¹ H ist;
R² H ist;
R³, R⁴, R⁵, R⁶ und R⁷ unabhängig aus H, Halogen, Alkylsulfonyl, N-Hydroxylsulfonamidyl, Perhalogenalkyl, Nitro, Aryl, Cyano, Alkoxy, Perhalogenalkoxy, Alkyl, substituiertem Aryloxy, Alkylsulfanyl, Alkylsulfinyl, Heterocycloalkyl, substituiertem Heterocycloalkyl, Dialkylamino, Cycloalkoxy, Cycloalkylsulfanyl, Arylsulfanyl und Arylsulfinyl ausgewählt sind, mit der Maßgabe, dass mindestens eines von R³, R⁴, R⁵, R⁶ und R⁷ anders als H ist; und dass mindestens eines von R³, R⁴, R⁵, R⁶ und R⁷ anders als Halogen ist; und
wobei R³, R⁵ und R⁷ H sind, eines von R⁴ und R⁶ H ist und das R⁴ oder R⁶, das nicht H ist, anders als N-Hydroxysulfonamidyl, Perhalogenalkyl oder Nitro ist.

9. Nitroxyldonator nach Anspruch 1 zur Verwendung in einem Verfahren, wie in einem der Ansprüche 1 bis 4 definiert, wobei der Nitroxyldonator ist:

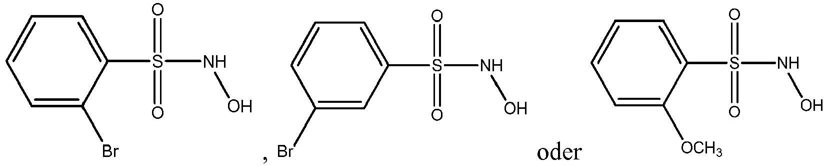

10. Nitroxyldonator zur Verwendung in einem Verfahren zum Behandeln von pulmonaler Hypertonie, welches Verfahren umfasst, an einen Säuger, der dessen bedarf, eine wirksame Menge von dem Nitroxyldonator zu verabreichen, wobei der Nitroxyldonator eine Verbindung der Formel (I) ist: 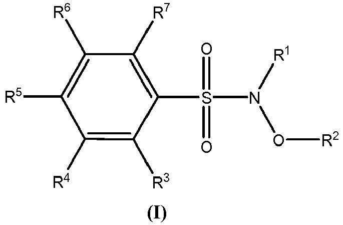 oder ein pharmazeutisch verträgliches Salz davon, wobei:
R¹ H ist;
R² H, Benzyl oder Tetrahydropyran-2-yl ist; und
R³, R⁴, R⁵, R⁶ und R⁷ unabhängig aus H, Cl, F, I, Br, SO₂CH₃, SO₂NHOH, CF₃, NO₂, Phenyl, CN, OCH₃, OCF₃, *t*-Butyl, O-Isopropyl, 4-Nitrophenyloxy, Propan-2-thiyl, Propan-2-sulfinyl, Morpholino, N-Methylpiperazino, Dimethylamino, Piperidino, Cyclohexyloxy, Cyclopentylsulfanyl, Phenylsulfanyl, Phenylsulfinyl, Carboxyl, Carboxylester, Acylamino und Sulfonylamino ausgewählt sind, mit der Maßgabe, dass mindestens eines von R³, R⁴, R⁵, R⁶ und R⁷ Sulfonylamino ist.

11. Nitroxyldonator zur Verwendung in einem Verfahren zum Behandeln von pulmonaler Hypertonie, welches Verfahren umfasst, an einen Säuger, der dessen bedarf, eine wirksame Menge von dem Nitroxyldonator zu verabreichen, wobei der Nitroxyldonator eine Verbindung der Formel (III) 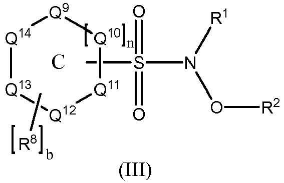 oder ein pharmazeutisch verträgliches Salz davon ist, wobei:
R¹ H ist;
R² H, Aralkyl oder Heterocyclyl ist;
Q⁹, Q¹⁰, Q¹¹, Q¹², Q¹³, Q¹⁴ und n derart definiert sind, dass Ring C Thiophen ist;
b 1 ist;
R⁸ Halogen, Alkylsulfonyl, *N*-Hydroxylsulfonamidyl, Perhalogenalkyl, Nitro, Aryl, Cyano, Alkoxy, Perhalogenalkoxy, Alkyl, substituiertes Aryloxy, Alkylsulfanyl, Alkylsulfinyl, Heterocycloalkyl, substituiertes Heterocycloalkyl, Dialkylamino, NH₂, OH, C(O)OH, C(O)OAlkyl, NHC(O)AlkylC(O)OH, C(O)NH₂, NHC(O)AlkylC(O)alkyl, NHC(O)AlkenylC(O)OH, NHC(O)NH₂, O-AlkylC(O)Oalkyl, NHC(O)Alkyl, C(=N-OH)NH₂, Cycloalkoxy, Cycloalkylsulfanyl, Arylsulfanyl oder Arylsulfinyl ist; und
Halogen F, Cl, Br oder I ist.

12. Nitroxyldonator zur Verwendung in einem Verfahren zum Behandeln von pulmonaler Hypertonie, welches Verfahren umfasst, an einen Säuger, der dessen bedarf, eine wirksame Menge von dem Nitroxyldonator zu verabreichen, wobei der Nitroxyldonator eine Verbindung der Formel (III) ist: 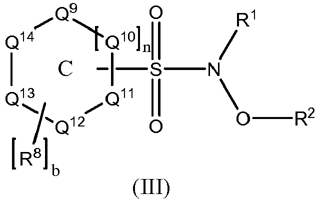 oder ein pharmazeutisch verträgliches Salz davon, wobei:
R¹ H ist;
R² H, Benzyl oder Tetrahydropyran-2-yl ist;
n 0 ist;
Q⁹, Q¹⁰, Q¹¹, Q¹², Q¹³ und Q¹⁴ unabhängig aus C, CH₂, CH, N, NR¹⁰, O und S ausgewählt sind, derart, dass C ein Thiophen, Isoxazol, Pyrazol, Pyrrol, Imidazol, Furan, Thiazol, Triazol, N-Methylimidazol oder Thiadiazol ist;
R¹⁰ H, Alkyl, Acyl oder Sulfonyl ist;
entweder (1) b 1 ist und R⁸ Halogen, Nitro, Alkyl oder Cyano ist oder (2) b 2 ist und jedes R⁸ unabhängig ein Halogen ist; und
Halogen F, Cl, Br oder I ist.

13. Nitroxyldonator zur Verwendung in einem Verfahren zum Behandeln von pulmonaler Hypertonie, welches Verfahren umfasst, an einen Säuger, der dessen bedarf, eine wirksame Menge von dem Nitroxyldonator zu verabreichen, wobei der Nitroxyldonator eine Verbindung der Formel (III) ist: 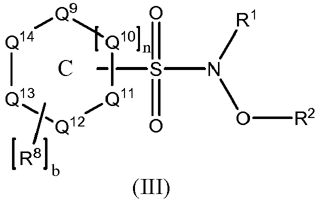 oder ein pharmazeutisch verträgliches Salz davon, wobei:
R¹ H ist;
R² H, Aralkyl oder Heterocyclyl ist;
n 0 ist;
Q⁹, Q¹⁰, Q¹¹, Q¹², Q¹³ und Q¹⁴ unabhängig aus C, CH₂, CH, N, NR¹⁰, O und S ausgewählt sind, derart, dass C ein Thiophen, Isoxazol, Pyrazol, Pyrrol, Imidazol, Furan, Thiazol, Triazol, N-Methylimidazol oder Thiadiazol ist;
R¹⁰ H, Alkyl, Acyl oder Sulfonyl ist;
b 1 ist und R⁸ Halogen, Nitro, Alkyl oder Cyano ist; und
Halogen F, Cl, Br oder I ist.

14. Nitroxyldonator zur Verwendung in einem Verfahren zum Behandeln von pulmonaler Hypertonie, welches Verfahren umfasst, an einen Säuger, der dessen bedarf, eine wirksame Menge von dem Nitroxyldonator zu verabreichen, wobei der Nitroxyldonator ist:

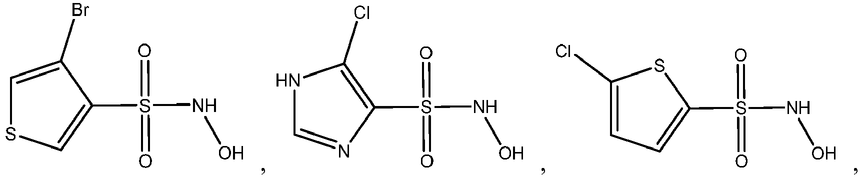

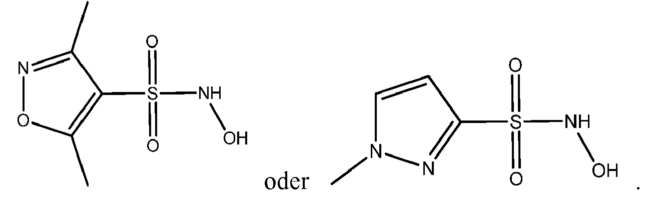

15. Nitroxyldonator zur Verwendung in einem Verfahren zum Behandeln von pulmonaler Hypertonie, welches Verfahren umfasst, an einen Säuger, der dessen bedarf, eine wirksame Menge von dem Nitroxyldonator zu verabreichen, wobei der Nitroxyldonator eine Verbindung der Formel (II) ist: 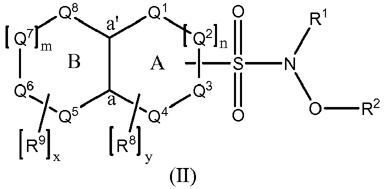 oder ein pharmazeutisch verträgliches Salz davon, wobei:
R¹ H ist;
R² H, Aralkyl oder Heterocyclyl ist;
m und n unabhängig eine ganze Zahl von 0 bis 1 sind;
A ein Cycloalkyl, Heterocycloalkyl, aromatischer oder heteroaromatischer Ring, enthaltend die Ringeinheiten Q¹, Q², Q³ und Q⁴, ist, welche mit den Kohlenstoffatomen an den Positionen a und a' zusammengenommen sind, um den Ring A zu bilden;
B ein Cycloalkyl, Heterocycloalkyl, aromatischer oder heteroaromatischer Ring, enthaltend die Ringeinheiten Q⁵, Q⁶, Q⁷ und Q⁸, ist, welche mit den Kohlenstoffatomen an den Positionen a und a' zusammengenommen sind, um den Ring B zu bilden;
Q¹, Q², Q³, Q⁴, Q⁵, Q⁶, Q⁷ and Q⁸ unabhängig aus C, CH₂, CH, N, NR¹⁰, O und S ausgewählt sind, mit der Maßgabe, dass, wenn die Ringe A und B Naphthalin bilden, x eine ganze Zahl von 1 bis 3 ist oder y eine ganze Zahl von 2 bis 3 ist;
x eine ganze Zahl von 0 bis 4 ist;
y eine ganze Zahl von 0 bis 3 ist;
jedes R⁸ und R⁹ unabhängig aus Cl, F, I, Br, SO₂CH₃, SO₂NHOH, CF₃, CH₃, NO₂, Phenyl, CN, OCH₃, OCF₃, *t*-Butyl, O-Isopropyl, 4-Nitrophenyloxy, Propan-2-thiyl, Propan-2-sulfinyl, Morpholino, N-Methyl-piperazino, Dimethylamino, Piperidino, Cyclohexyloxy, Cyclopentylsulfanyl, Phenylsulfanyl und Phenylsulfinyl ausgewählt ist; und
R¹⁰ H, Alkyl, Acyl oder Sulfonyl ist.

16. Nitroxyldonator nach Anspruch 15 zur Verwendung in einem Verfahren, wie in Anspruch 15 definiert, wobei der Nitroxyldonator ist:

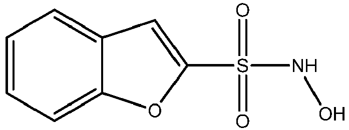

## Revendications

1. Donneur de nitroxyle pour une utilisation dans une méthode de traitement d'une hypertension pulmonaire, laquelle méthode comprend l'administration à un mammifère en ayant besoin d'une quantité efficace du donneur de nitroxyle, où le donneur de nitroxyle est un composé de la formule I: 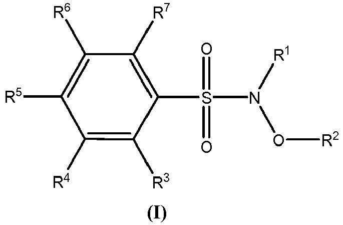 ou un sel pharmaceutiquement acceptable de celui-ci, dans lequel:
R¹ est H;
R² est H;
R³, R⁴, R⁵, R⁶ et R⁷ sont indépendamment choisis parmi H, un halo, un alkylsulfonyle, un *N*-hydroxylsulfonamidyle, un perhaloalkyle, un nitro, un aryle, un cyano, un alcoxy, un perhaloalcoxy, un alkyle, un aryloxy substitué, un alkylsulfanyle, un alkylsulfinyle, un hétérocycloalkyle, un hétérocycloalkyle substitué, un dialkylamino, un cycloalcoxy, un cycloalkylsulfanyle, un arylsulfanyle et un arylsulfinyle.

2. Donneur de nitroxyle selon la revendication 1 pour une utilisation dans une méthode telle que définie dans la revendication 1, où la quantité efficace est inférieure à environ 1 g/kg/d et/ou dans lequel le mammifère est un humain.

3. Donneur de nitroxyle selon la revendication 1 pour une utilisation dans une méthode telle que définie dans la revendication 1 ou 2, où le composé est administré par voie intraveineuse, orale ou par inhalation.

4. Donneur de nitroxyle selon la revendication 1 pour une utilisation dans une méthode telle que définie dans l'une quelconque des revendications 1 à 3, où l'hypertension pulmonaire est une hypertension artérielle pulmonaire, une hypertension pulmonaire thromboembolique chronique ou une hypertension pulmonaire due à une maladie du coeur gauche, où la maladie du coeur gauche est optionnellement une insuffisance du coeur gauche et où l'insuffisance du coeur gauche est optionnellement (i) une insuffisance cardiaque systolique, (ii) une insuffisance cardiaque diastolique, ou (iii) chronique ou décompensée de façon aiguë.

5. Donneur de nitroxyle selon la revendication 1 pour une utilisation dans une méthode telle que définie dans l'une quelconque des revendications 1 à 4, dans lequel:
(1) un de R³ et R⁷ est autre que H; au moins un de R³, R⁴, R⁵, R⁶ et R⁷ est autre qu'un halo; lorsqu'un de R³ ou R⁷ est un halo et que le R³ ou R⁷ qui n'est pas un halo est H et qu'un de R⁴ ou R⁶ est un halo et que le R⁴ ou R⁶ qui n'est pas un halo est H, R⁵ est autre qu'un halo ou un hydrogène; lorsque R⁴, R⁵ et R⁶ sont H et qu'un de R³ et R⁷ est H, le R³ ou R⁷ qui n'est pas H est autre qu'un nitro ou un alkyle; et lorsque R⁴ et R⁶ sont H et que R³ et R⁷ sont des alkyles, R⁵ est autre qu'un alkyle; ou
(2) R³ est un halo, un alkylsulfonyle, un perhaloalkyle, un alkyle possédant de 1 à 4 atomes de carbone, un nitro ou un cyano; ou
(3) R³ est un halo, un alkylsulfonyle, un perhaloalkyle, un alkyle possédant de 1 à 4 atomes de carbone, un nitro ou un cyano; et au moins trois de R⁴, R⁵, R⁶ et R⁷ sont H; ou
(4) R³ est un halo, un alkylsulfonyle, un perhaloalkyle, un alkyle possédant de 1 à 4 atomes de carbone, un nitro ou un cyano; et R⁴, R⁵, R⁶ et R⁷ sont H; ou
(5) R³ est un halo, un méthylsulfonyle, un perfluorométhyle, un perfluorométhoxy, un isopropyle, un nitro ou un cyano; ou
(6) R³ est un halo, un méthylsulfonyle, un perfluorométhyle, un perfluorométhoxy, un isopropyle, un nitro ou un cyano; et au moins trois de R⁴, R⁵, R⁶ et R⁷ sont H; ou
(7) R³ est un halo, un méthylsulfonyle, un perfluorométhyle, un perfluorométhoxy, un isopropyle, un nitro ou un cyano; et R⁴, R⁵, R⁶ et R⁷ sont H.

6. Donneur de nitroxyle selon la revendication 1 pour une utilisation dans une méthode telle que définie dans l'une quelconque des revendications 1 à 4, où le donneur de nitroxyle est un composé choisi parmi:
le 2,6-dichloro-*N*-hydroxybenzènesulfonamide;
le 2-bromo-4-fluoro-*N*-hydroxybenzènesulfonamide;
le 2,5-di-trifluorométhyl-*N*-hydroxybenzènesulfonamide;
le 2-chloro-4-fluoro-*N*-hydroxybenzènesulfonamide;
le 2,3-dichloro-*N*-hydroxybenzènesulfonamide,
le 2-chloro-4-bromo-*N*-hydroxybenzènesulfonamide;
le 2-nitro-4-trifluorométhyl-*N*-hydroxybenzènesulfonamide;
le 2-iodo-*N*-hydroxybenzènesulfonamide;
le *N*-hydroxy-2-méthanesulfonylbenzènesulfonamide;
le 2,4-di-bromo-*N*-hydroxybenzènesulfonamide;
le 2-chloro-4-trifluorométhyl-*N*-hydroxybenzènesulfonamide;
le 2,4,6-tri-isopropyl-*N*-hydroxybenzènesulfonamide;
le 2,4-di-fluoro-*N*-hydroxybenzènesulfonamide;
le 2-fluoro-*N*-hydroxybenzènesulfonamide;
le 2-bromo-*N*-hydroxybenzènesulfonamide;
le 2-(trifluorométhyl)-*N*-hydroxybenzènesulfonamide;
le N-hydroxy-2-phénylbenzènesulfonamide; et
des sels pharmaceutiquement acceptables de ceux-ci.

7. Donneur de nitroxyle selon la revendication 1 pour une utilisation dans une méthode telle que définie dans l'une quelconque des revendications 1 à 4, où le composé est le 2-iodo-N-hydroxybenzènesulfonamide, le *N*-hydroxy-2-méthanesulfonylbenzènesulfonamide, le 2-fluoro-N-hydroxybenzènesulfonamide, le 2-chloro-*N*-hydroxybenzènesulfonamide, le 2-bromo-N-hydroxybenzènesulfonamide ou le 2-(trifluorométhyl)-*N*-hydroxybenzènesulfonamide.

8. Donneur de nitroxyle selon la revendication 1 pour une utilisation dans une méthode telle que définie dans l'une quelconque des revendications 1 à 4, où le donneur de nitroxyle est un composé de la formule (I): 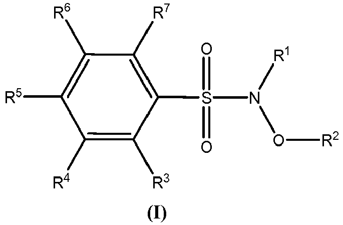 ou un sel pharmaceutiquement acceptable de celui-ci, dans lequel:
R¹ est H;
R² est H;
R³, R⁴, R⁵, R⁶ et R⁷ sont indépendamment choisis parmi H, un halo, un alkylsulfonyle, un N-hydroxylsulfonamidyle, un perhaloalkyle, un nitro, un aryle, un cyano, un alcoxy, un perhaloalcoxy, un alkyle, un aryloxy substitué, un alkylsulfanyle, un alkylsulfinyle, un hétérocycloalkyle, un hétérocycloalkyle substitué, un dialkylamino, un cycloalcoxy, un cycloalkylsulfanyle, un arylsulfanyle et un arylsulfinyle, à condition que au moins un de R³, R⁴, R⁵, R⁶ et R⁷ soit autre que H; et que au moins un de R³, R⁴, R⁵, R⁶ et R⁷ soit autre qu'un halo; et
dans lequel R³, R⁵ et R⁷ sont H, un de R⁴ et R⁶ est H et le R⁴ ou R⁶ qui n'est pas H est autre qu'un N-hydroxysulfonamidyle, un perhaloalkyle ou un nitro.

9. Donneur de nitroxyle selon la revendication 1 pour une utilisation dans une méthode telle que définie dans l'une quelconque des revendications 1 à 4, où le donneur de nitroxyle est:

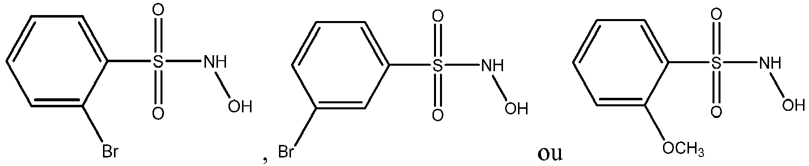

10. Donneur de nitroxyle pour une utilisation dans une méthode de traitement d'une hypertension pulmonaire, laquelle méthode comprend l'administration à un mammifère en ayant besoin d'une quantité efficace du donneur de nitroxyle, où le donneur de nitroxyle est un composé de la formule (I): 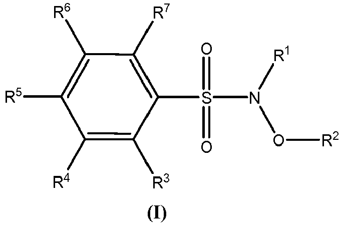 ou un sel pharmaceutiquement acceptable de celui-ci, dans lequel:
R¹ est H;
R² est H, un benzyle ou un tétrahydropyran-2-yle; et
R³, R⁴, R⁵, R⁶ et R⁷ sont indépendamment choisis parmi H, Cl, F, I, Br, SO₂CH₃, SO₂NHOH, CF₃, NO₂, un phényle, CN, OCH₃, OCF₃, un t-butyle, un O-isopropyle, un 4-nitrophényloxy, un propane-2-thiyle, un propane-2-sulfinyle, un morpholino, un N-méthyl-pipérazino, un diméthylamino, un pipéridino, un cyclohexyloxy, un cyclopentylsulfanyle, un phénylsulfanyle, un phénylsulfinyle, un carboxyle, un ester de carboxyle, un acylamino et un sulfonylamino, à condition que au moins un de R³, R⁴, R⁵, R⁶ et R⁷ soit un sulfonylamino.

11. Donneur de nitroxyle pour une utilisation dans une méthode de traitement d'une hypertension pulmonaire, laquelle méthode comprend l'administration à un mammifère en ayant besoin d'une quantité efficace du donneur de nitroxyle, où le donneur de nitroxyle est un composé de la formule (III): 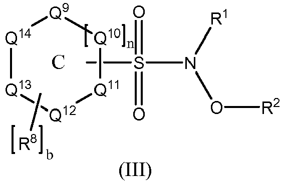 ou un sel pharmaceutiquement acceptable de celui-ci, dans lequel:
R¹ est H;
R² est H, un aralkyle ou un hétérocyclyle;
Q⁹, Q¹⁰, Q¹¹, Q¹², Q¹³, Q¹⁴ et n sont définis de sorte que le cycle C est un thiophène;
b est 1;
R⁸ est un halo, un alkylsulfonyle, un N-hydroxylsulfonamidyle, un perhaloalkyle, un nitro, un aryle, un cyano, un alcoxy, un perhaloalcoxy, un alkyle, un aryloxy substitué, un alkylsulfanyle, un alkylsulfinyle, un hétérocycloalkyle, un hétérocycloalkyle substitué, un dialkylamino, NH₂, OH, C(O)OH, un C(O)Oalkyle, un NHC(O)alkylC(O)OH, C(O)NH₂, un NHC(O)alkylC(O)alkyle, un NHC(O)alcénylC(O)OH, NHC(O)NH₂, un O-alkylC(O)Oalkyle, un NHC(O)alkyle, C(=N-OH)NH₂, un cycloalcoxy, un cycloalkylsulfanyle, un arylsulfanyle ou un arylsulfinyle; et
l'halo est F, Cl, Br ou I.

12. Donneur de nitroxyle pour une utilisation dans une méthode de traitement d'une hypertension pulmonaire, laquelle méthode comprend l'administration à un mammifère en ayant besoin d'une quantité efficace du donneur de nitroxyle, où le donneur de nitroxyle est un composé de la formule (III): 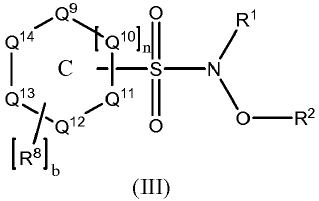 ou un sel pharmaceutiquement acceptable de celui-ci, dans lequel:
R¹ est H;
R² est H, un benzyle ou un tétrahydropyran-2-yle;
n est 0;
Q⁹, Q¹⁰, Q¹¹, Q¹², Q¹³ et Q¹⁴ sont indépendamment choisis parmi C, CH₂, CH, N, NR¹⁰, O et S de sorte que C est un thiophène, un isoxazole, un pyrazole, un pyrrole, un imidazole, un furane, un thiazole, un triazole, un N-méthylimidazole ou un thiadiazole;
R¹⁰ est H, un alkyle, un acyle ou un sulfonyle;
soit (1) b est 1 et R⁸ est un halo, un nitro, un alkyle ou un cyano, soit (2) b est 2 et chaque R⁸ est indépendamment un halo; et
l'halo est F, Cl, Br ou I.

13. Donneur de nitroxyle pour une utilisation dans une méthode de traitement d'une hypertension pulmonaire, laquelle méthode comprend l'administration à un mammifère en ayant besoin d'une quantité efficace du donneur de nitroxyle, où le donneur de nitroxyle est un composé de la formule (III): 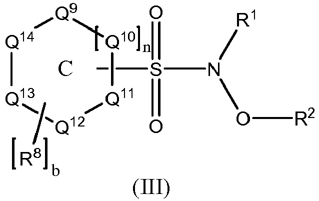 ou un sel pharmaceutiquement acceptable de celui-ci dans lequel:
R¹ est H;
R² est H, un aralkyle ou un hétérocyclyle;
n est 0;
Q⁹, Q¹⁰, Q¹¹, Q¹², Q¹³ et Q¹⁴ sont indépendamment choisis parmi C, CH₂, CH, N, NR¹⁰, O et S de sorte que C est un thiophène, un isoxazole, un pyrazole, un pyrrole, un imidazole, un furane, un thiazole, un triazole, un N-méthylimidazole ou un thiadiazole;
R¹⁰ est H, un alkyle, un acyle ou un sulfonyle;
b est 1 et R⁸ est un halo, un nitro, un alkyle ou un cyano; et
l'halo est F, Cl, Br ou I.

14. Donneur de nitroxyle pour une utilisation dans une méthode de traitement d'une hypertension pulmonaire, laquelle méthode comprend l'administration à un mammifère en ayant besoin d'une quantité efficace du donneur de nitroxyle, où le donneur de nitroxyle est:

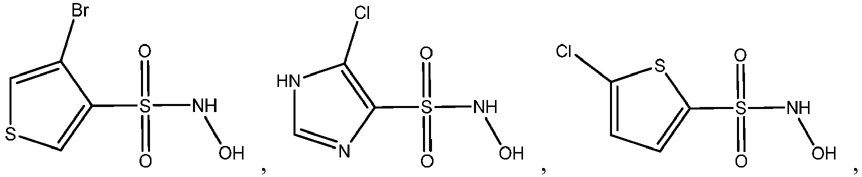

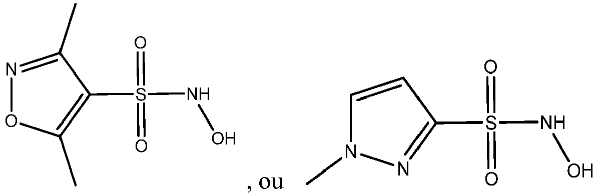

15. Donneur de nitroxyle pour une utilisation dans une méthode de traitement d'une hypertension pulmonaire, laquelle méthode comprend l'administration à un mammifère en ayant besoin d'une quantité efficace du donneur de nitroxyle, où le donneur de nitroxyle est un composé de la formule (II): 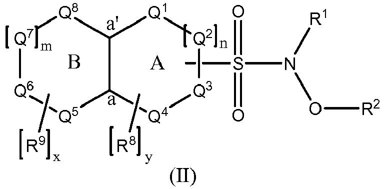 ou un sel pharmaceutiquement acceptable de celui-ci, dans lequel:
R¹ est H;
R² est H, un aralkyle ou un hétérocyclyle;
m et n sont indépendamment un nombre entier de 0 à 1;
A est un cycloalkyle, un hétérocycloalkyle, un cycle aromatique ou hétéroaromatique contentant des fractions de cycle Q¹, Q², Q³ et Q⁴, qui sont prises ensemble avec les carbones aux positions a et a' pour former le cycle A;
B est un cycloalkyle, un hétérocycloalkyle, un cycle aromatique ou hétéroaromatique contenant des fractions de cycle Q⁵, Q⁶, Q⁷ et Q⁸, qui sont prises ensemble avec les carbones aux positions a et a' pour former le cycle B;
Q¹, Q², Q³, Q⁴, Q⁵, Q⁶, Q⁷ et Q⁸ sont indépendamment choisies parmi C, CH₂, CH, N, NR¹⁰, O et S, à condition que lorsque les cycles A et B forment un naphtalène, x soit un nombre entier de 1 à 3 ou y soit un nombre entier de 2 à 3;
x est un nombre entier de 0 à 4;
y est un nombre entier de 0 à 3;
chaque R⁸ et R⁹ est indépendamment choisi parmi Cl, F, I, Br, SO₂CH₃, SO₂NHOH, CF₃, CH₃, NO₂, un phényle, CN, OCH₃, OCF₃, un t-butyle, un O-isopropyle, un 4-nitrophényloxy, un propane-2-thiyle, un propane-2-sulfinyle, un morpholino, un N-méthyl-pipérazino, un diméthylamino, un pipéridino, un cyclohexyloxy, un cyclopentylsulfanyle, un phénylsulfanyle et un phénylsulfinyle, et
R¹⁰ est H, un alkyle, un acyle ou un sulfonyle.

16. Donneur de nitroxyle selon la revendication 15 pour une utilisation dans une méthode telle que définie dans la revendication 15, où le donneur de nitroxyle est:
